# FASCICULE DE BREVET EUROPEEN

(11) **EP 0 754 231 B1**
(45) Date de publication et mention de la délivrance du brevet: **21.06.2006**
(21) Numéro de dépôt: 95916721.4
(22) Date de dépôt: 06.04.1995
(51) Int. Cl.: C12N 15/45, C12N 15/31, C07K 14/135, C07K 14/26, C07K 14/765, A61K 39/155, A61K 47/48

(54) **FRAGMENT PEPTIDIQUE DE LA PROTEINE G DU VIRUS RESPIRATOIRE SYNCYTIAL, AGENT IMMUNOGENE, COMPOSITION PHARMACEUTIQUE LE CONTENANT ET PROCEDE DE PREPARATION**
PEPTIDFRAGMENT DES G-PROTEINS DES RESPIRATORISCHEN SYNCYTIALVIRUS, IMMUNOGENE VERBINDUNG UND PHARMAZEUTISCHE ZUSAMMENSETZUNG, DIE ES ENTHALTEN, UND HERSTELLUNGSVERFAHREN
PEPTIDE FRAGMENT OF THE RESPIRATORY SYNCYTIAL VIRUS G PROTEIN, IMMUNOGENIC AGENT, PHARMACEUTICAL COMPOSITION CONTAINING SAME, AND PREPARATION METHOD

(30) Priorité: 06.04.1994 FR 9404009
(43) Date de publication de la demande: 22.01.1997
(60) Demande divisionnaire: 00126606.3
(73) Titulaire: PIERRE FABRE MEDICAMENT, 92100 Boulogne (FR)
(72) Inventeur: BINZ, Hans, F-74160 Beaumont (FR); N'GUYEN, Ngoc, Thien, F-74160 Saint-Julien-en-Genevois (FR); BAUSSANT, Thierry, F-01200 Bellegarde (FR); TRUDEL, Michel, Lorraine, Québec J6Z 3Y3 (CA)
(74) Mandataire: Ahner, Francis
(86) Numéro de dépôt international: PCT/FR1995/000444
(87) Numéro de publication internationale: WO 1995/027787

(56) Documents cités:
- EP-A- 0 355 737
- WO-A-89/05823
- WO-A-92/04375
- WO-A-92/20805
- WO-A-93/14207
- PROC. NATL. ACAD. SCI. U. S. A. (1987), 84(18), 6572-6 CODEN: PNASA6;ISSN: 0027-8424, 1987 NORRBY, ERLING ET AL 'Site-directed serology with synthetic peptides representing the large glycoprotein G of respiratory syncytial virus'
- JOURNAL OF GENERAL MICROBIOLOGY, vol. 137, no. 8, Août 1991 pages 1911-1921, J.G. LAWRENCE ET AL. 'Molecular and evolutionary relationship among enteric bacteria'
- JOURNAL OF VIROLOGY, vol. 63, no. 2, Février 1989 pages 925-932, B. GARCIA-BARRENO ET AL. 'Marked Differences in the Antigenic Structure of Human Respiratory Syncytical Virus F and G Glycoproteins'

## Description

La présente invention se rapporte à virus respiratoire syncytial (VRS) à une protéine OmpA adjuvante d'immunité extraite de Klebsiella pneumoniae, à des compositions contenant des polypeptides immunogènes issus d' associés à une telle protéine adjuvante, ainsi qu'à leur procédé de préparation.

Le virus respiratoire syncytial (VRS) est la cause la plus fréquente de maladies respiratoires chez le nouveau-né : bronchopneumopathies (bronchiolites). L'OMS estime chaque année 50 millions de cas atteints du VRS, dont 160 000 décès dans le monde entier. Il existe deux sous groupes du virus (sous groupes A et B).

Le VRS est classé dans la famille des Paramyxoviridae, genre pneumovirus comportant un génome ARN non segmenté, de polarité négative, codant pour 10 protéines spécifiques.

Il n'existe pas actuellement de vaccin disponible, contre le VRS. Les vaccins à virus inactivé se sont montrés inefficaces et ont même parfois aggravé les infections des nourrissons. Dans les années 60, les tentatives de vaccination avec le VRS inactivé à la formaline ont conduit à l'échec : au lieu de conférer une protection lors de la réinfection due au VRS, le vaccin a eu pour effet d'aggraver la maladie chez l'enfant.

La demande WO 87/04185 a proposé d'utiliser des protéines structurales du VRS en vue d'un vaccin, comme les protéines d'enveloppe appelées protéine F (protéine de fusion) ou protéine G, une glycoprotéine de 22 Kd, une protéine de 9,5 Kd, ou la protéine majeure de capside (protéine N).

La demande WO 89/02935 décrit les propriétés de protection de la protéine F entière du VRS, éventuellement modifiées sous forme monomériques ou désacétylée.

Une série de fragments de la protéine F a été clonée en vue de rechercher leurs propriétés neutralisantes.

On peut citer le document Norrby et al. (Proc. Natl.Acad.Sci USA, 1987 84 (18), 6572-76) qui décrit certains fragments de la protéine G du VRS (peptides 12 et 15 de ce document) comme région la plus immunogène de la protéine G.

On peut également citer le document Lawrence et al. (J. of General Microbiology, 1991, 137 (8), 1911-21) relatif à la classification des entérobactéries qui décrit la séquence nucléique partielle de 18 locus correspondant à la séquence codant pour la protéine de membrane externe (OmpA) de 18 entérobactéries dont Klebsietta pneumoniae.

Toutefois les vaccins immunitaires testés à ce jour se sont montrés inefficaces ou ont induit une pathologie pulmonaire (bronchiolite ou péribronchite).

A l'heure actuelle il n'existe pas de traitement de fond des infections dues au VRS.

Les infections au VRS des voies aériennes supérieures : le traitement repose essentiellement sur les médications symptomatiques identiques à celles des autres infections virales.

Les infections au VRS des voies aériennes inférieures : le traitement chez les nourrissons repose sur le maintien d'une hydratation correcte, l'aspiration des sécrétions et l'administration d'oxygène si besoin. Un effet positif a été observé avec la ribavirine, nucléotide actif in vitro contre le VRS.

La présente invention décrit un polypeptide utile notamment dans la production d'immunogène, porté par la séquence peptidique comprise entre les résidus d'acides aminés 130 et 230 de la séquence de la protéine G du virus respiratoire syncytial, ou par une séquence présentant au moins 80% d'homologie avec ladite séquence peptidique. Cette séquence diffère légèrement pour les sous-groupes A et B du VRS humain, ou pour le VRS bovin. L'invention comprend les séquences provenant des VRS humain sous-groupe A et B, ou bovin

La protéine G est une glycoprotéine d'enveloppe du VRS, de poids moléculaire compris entre 84 et 90 Kd, pauvre en méthionine.

La Demanderesse a mis en évidence que la séquence comprise entre les acides aminés 130 et 230 de la protéine G naturelle est particulièrement appropriée pour induire une protection efficace contre l'infection par le VRS. L' invention comprend les séquences provenant des VRS humain sous-groupe A ou B, ou bovin.

La présente invention décrit particulièrement des polypeptides, utiles notamment comme élément d'immunogène compris dans le précédent et qui comportent la séquence peptidique comprise entre les résidus aminoacides numérotés 174 et 187 de la protéine G du VRS (humain, sous-groupes A et B, ou bovin) ou une séquence présentant au moins 80% d'homologie avec la séquence correspondante.

D'autres séquences peptidiques adaptées à la préparation d'un immunogène comprises dans ladite séquence de la protéine G du VRS sont constituées par la séquence comprise entre les résidus aminoacides numérotés 171 et 187 de la protéine G du VRS humain ou bovin, ou une séquence présentant au moins 8096 d'homologie avec la séquence correspondante. D'autres peptides d'intérêt sont portées par la séquence comprise entre les nucléotides numérotés 158 et 190 de la protéine G du VRS ou une séquence présentant au moins 8096 d'homologie avec la séquence correspondante.

L' invention décrit également des peptides utiles pour la préparation d'un immunogene et qui présentent une séquence correspondant à la séquence comprise entre les résidus aminoacides numérotés 140 et 200 de la protéine G du VRS humain ou bovin, ou une séquence présentant au moins 80% d'homologie avec la séquence correspondante. Des séquences débutant à l'aminoacide 140 de ladite protéine G du VRS et dont l'extrémité C-terminale correspond respectivement à l'aminoacide 198, 196, 194, 192, ou 190 ainsi, que des séquences présentant au moins 80% d'homologie avec les séquences portées par ces fragments sont particulièrement avantageuses.

Parmi les variants des séquences précédentes, il faut citer les polypeptides qui comportent une séquence dans laquelle :
a) l'acide aminé Cys en positions 173 et/ou 186 a été remplacé par un aminoacide ne formant pas de pont disulfure en particulier la serine, et/ou
b) les acides aminés en positions 176 et 182 sont susceptibles de former un pont covalent autre qu'un pont disulfure notamment l'acide aspartique et l'ornithine.

Ainsi, la séquence polypeptidique 130-230 du VRS sous-groupe A peut étn utilisée complète, sous sa forme native. Cette séquence correspond à la séquence notée Seq id n° 1 (ou G2A).

De même, on peut utiliser la séquence polypeptidique complète 130-230 du VRS sous groupe B, sous sa forme native. Cette séquence correspond à la séquence notée Seq id n° 2 (G2B).

La sequence id n° 1 sera notée G2A dans la suite de la demande.

La séquence id n° 2 sera notée G2B dans la suite de la demande.

Des séquences présentant au moins 80% d'homologie avec G2A ou G2B sont également appropriées.

La séquence comprise entre les acides aminés 130 et 230, peut être modifiée par le remplacement des résidus cystéine en positions 173 et 186 par des résidus sérine pour obtenir un peptide conservant de bonnes propriétés immunogènes, grâce au maintien de la boucle formée par les résidus Cys en positions 176 et 182. Les séquences en acides aminés et nucléotides de ce polypeptide pour le sous-groupe A sont représentées sur la seq id n° 3 (G2AδCys).

Pour le sous-groupe B. les séquences en acides aminés et en nucléotides sont représentées sur la seq id n° 4 (G2BδCys).

Les séquences peptidiques seront notées G2AδCys et G2BδCys.

On décrit également dans la présente demande un polypeptide utile pour la préparation d'immunogène, consistant en la séquence peptidique comprise entre les résidus aminoacides numérotés 174 et 187 de la protéine G du VRS ou une séquence présentant au moins 80% d'homologie avec ladite séquence peptidique.

Dans cette dernière séquence le peptide 174-187 sous-groupe A peut présenter la séquence :
Seq id n° 5 :
   Ser Ile Cys Ser Asn Asn Pro Thr Cys Trp Ala Ile Cys Lys.
   Le peptide 174-187 sous-groupe B peut présenter la séquence :
Seq id n° 6 :
   Ser-Ile-Cys-Gly-Asn-Asn-Gln-Leu-Cys-Lys-Ser-Ile-Cys-Lys.
   Le résidu Cys en position 186 peut également être remplacé par un résidu sérine, de manière à obtenir la séquence suivante :
Seq id n° 7 pour le sous-groupe A :
   Ser Ile Cys Ser Asn Asn Pro Thr Cys Trp Ala Ile Ser Lys.
Seq id n° 8 pour le sous-groupe B :
   Ser-Ile-Cys-Gly-Asn-Asn-Gln-Leu-Cys-Lys-Ser-Ile-Ser-Lys.

Dans la séquence comprise entre les résidus 174 et 187 du peptide immunogène, les résidus aminoacides en positions 176 et 182 peuvent être respectivement remplacés par un acide aspartique et une ornithine, de manière à obtenir l'une des séquences suivantes :
Seq id n° 9 pour le sous-groupe A :
   Ser Ile Asp Ser Asn Asn Pro Thr Orn Trp Ala Ile Cys Lys
Seq id n° 10 pour le sous-groupe B
   Ser-Ile-Asp-Gly-Asn-Asn-Gln-Leu-Orn-Lys-Ser-Ile-Cys-Lys.
Seq id n° 11 pour le sous-groupe A :
   Ser Ile Asp Ser Asn Asn Pro Thr Orn Trp Ala lie Ser Lys.
Seq id n° 12 pour le sous-groupe B :
   Ser-Ile-Asp-Gly-Asn-Asn-Gln-Leu-Orn-Lys-Ser-Ile-Ser-Lys.

Le maintien des propriétés immunogènes est obtenu grâce au remplacement du pont disulfure (entre les Cys naturelles) par un pont amide entre les positions 176 et 182.

D'autres séquences de polypeptide immunogènes figurent en annexe de la présente demande sous les désignations SEQ ID N°14 à SEQ ID N°73.

L'invention décrit également un polypeptide utilisable comme agent immunogène présentant l'une des séquences précédentes et qui comporte en outre au moins un résidu cystéine en position N-terminale ou C- terminale.

L'invention décrit également un polypeptide qui consiste en la séquence peptidique comprise entre les résidus aminoacides numérotés 130 et 230 de la séquence de la protéine G du VRS sous-groupe A et sous-groupe B, ou en une séquence présentant 8096 d'homologie avec ladite séquence peptidique et qui est sous forme d'une protéine de fusion avec le récepteur de la serumalbumine humaine, nommée BBG2AδC ou BBG2BδC, ou une autre protéine de liaison. La séquence de la protéine BB complète figure en annexe (Seq ID n° 74).

On peut citer également les variants par exemple glycosylés ou sulfatés des différents peptides, que ces fonctions soient naturelles ou non.

Les polypeptides peuvent être préparés par synthèse peptidique ou par les techniques d'ADN recombinant, qui sont connues de l'homme du métier.

En particulier, les séquences du gène codant pour l'épitope d'environ 100 acides aminés peuvent être préparées par assemblage de gènes en phase solide, et la protéine correspondante exprimée par exemple dans E coli par voie intracellulaire.

Les séquences nucléotidiques (ARN ou ADN) codant pour les protéines ou les polypeptides définis ci-dessus peuvent être également citées.

La présente invention a pour objet une protéine isolée caractérisée en ce qu'elle est la protéine OmpA de la membrane externe de Kbelsiella pneumoniae de séquence SEQ ID N° 13.

Un autre objet de l'invention est un conjungué immunogène qui comporte un polypeptide tel que défini précédemment couplé à

la protéine porteuse de type OmpA de la membrane externe de bactérie du genre Klebsiella selon l'invention.

La Demanderesse a pu montrer qu'alors que les variants de la séquence 174-187 de la protéine G du VRS sont faiblement immunogènes, leur couplage avec une telle protéine induit une réponse immunitaire spécifique.

L'intensité de la réponse immunitaire a été comparée avec celle obtenue avec des adjuvants classiques, tel que le couplage au porteur KLH (keyhole limpet hemocyanin) coadministré avec l'adjuvant de Freund, ou le couplage à la protéine porteuse TT (tetanus toxoid).

Des résultats particulièrement avantageux sont obtenus pour des compositions contenant un polypeptide immunogène de URS tel que décrit précédemment couplé à la protéine p40 de Klebsiella pneumoniae de séquence peptidique notée Seq id n° 13.

La séquence nucléotidique (ADN ou ARN) codant pour la protéine de séquence id n° 13 est comprise dans l'invention.

Le polypeptide immunogène peut être couplé à la protéine adjuvante d'immunité par des méthodes connues de l'homme du métier telles que :
- Glutaraldéhyde
- Carbodiimide (ex : EDC: 1-(3diméthylaminopropyl)-3-éthylcarbodiimide).
- Bis imido esters (ex : diméthyladipimidate).
- N-hydroxysuccinimidyl esters (ex : disuccinimidyl subérate).
- Pour les peptides comportant une cystéine supplémentaire en position N terminale ou C terminale :
   * Maléimido-N-hydroxysuccinimide esters (ex : MBS : maléimido benzoyl-N-hydroxy-succinimide ester).
   * N- succinimidyl Bromoacétate.

Le polypeptide peut être conjugué à la protéine porteuse par une protéine de liaison, par exemple le récepteur de la sérumalbumine humaine (BB).

Selon un autre aspect, l'invention a également pour objet un procédé de préparation d'un peptide conjugué entrant dans une composition utile pour prévention ou traitement des affections à VRS, caractérisé en ce que :
a) on précipite les lipopolysaccharides de membranes de bactéries du genre Klebsiella, en présence d'un sel de cation divalent et de détergents, pour récupérer les protéines membranaires totales dans le surnageant.
b) on soumet les protéines à une chromatographie par échange d'anions pour séparer la fraction contenant la protéine adjuvante d'immunité,
c) on concentre la fraction contenant la protéine adjuvante d'immunité,
d) on conjugue la protéine adjuvante d'immunité avec un polypeptide immunogène tel que définis ci-dessus pour former un conjugué soluble, ledit polypeptide immunogène comprenant la séquence aa 174-187 de la séquence aa 130-230 de la protéine G du VRS humain de type A ou B, ou du VRS bovin, et dans lequel polypeptide immunogène, l'amino acid cys en position 173 et/ou 186 peut être une sérine.

Le sel de cation divalent utilisé dans l'étape a) est de préférence un sel de calcium ou de magnésium. Après centrifugation, les protéines du surnageant peuvent être récupérées avec un bon rendement par deux précipitations à l'éthanol.

Les protéines membranaires, après remise en suspension, sont séparées sur une colonne échangeuse d'anions, utilisable en conditions industrielles. Ce support chromatographique est très stable et compatible avec les traitements de dépyrogénation drastiques, ce qui n'était pas le cas des supports chromatographiques déjà décrits. D'autre part, l'élution de la protéine peut être réalisée en conditions isocratiques et non par application d'un gradient de NaCl (comme décrit précédemment), ce qui est particulièrement avantageux en conditions industrielles.

Selon un mode de réalisation préféré, après l'étape c), on procède à une seconde étape de chromatographie, sur échangeur de cations, et on récupère les fractions contenant la protéine adjuvante, qui sont concentrées. Cette étape supplémentaire permet une meilleure élimination des lipopolysaccharides. La protéine adjuvante est ensuite conjuguée avec un polypeptide immunogène selon l'invention

Selon un autre aspect, l'invention décrit une composition utile pour la prévention et/ou le traitement des affections provoquées par le VRS, caractérisée en ce qu'elle contient un polypeptide caractérisé ci-avant.

Plus particulièrement les compositions contiennent en outre des excipients pharmaceutiquement acceptables adaptés à l'administration par voie injectable.

En effet, la Demanderesse a mis en évidence que l'injection de telles compositions entraine une protection, non par un effet neutralisant, mais par une réponse immunitaire systémique de l'organisme.

Les réponses humorales et cellulaire (IgM, IgG, IgA et cellules T) sont provoquées par le produit qui induit également une protection à long terme et une mémoire immunologique contre les VRS sous groupes a et b.

En vue de l'administration des compositions vaccinales par voie sous-cutanée, il est souhaitable de disposer de conjugué soluble, ce qui est difficile par les méthodes conventionnelles.

L'invention décrit également un procédé de préparation d'un conjugué entre un peptide immunogène et une protéine de membrane de Klebsiella, en particulier la protéine p40 de K. pneumoniae, dans lequel le couplage est effectué en présence de glutaraldéhyde à des concentrations inférieures ou égales à 0,05%.

Ce procédé de couplage diminue considérablement les concentrations en glutaraldéhyde en comparaison de celles habituellement utilisées (2 fois 0,01% au lieu de 196 environ) ; le glutaraldéhyde est ajouté en 2 fois sur une période de cinq jours alors que les protocoles décrits mentionnent des temps de 24 heures.

Ces modifications ont permis l'obtention d'un conjugué soluble, sous une forme adaptée à l'administration sous cutanée.

Les protocoles usuels (concentrations en glutaraldéhyde plus élevées et temps courts) se traduisent par la formation d'un gel dense (dû à des réactions de conjugaison P40-P40, très probablement), forme impropre à l'administration et à la manipulation en général.

Le peptide conjugué peut être congelé et utilisé tel quel ou lyophilisé.

Les exemples qui suivent sont destinés à illustrer l'invention sans aucunement en limiter la portée.

Dans ces exemples on se référera aux figures suivantes :
- Figure 1 : intensité de la réponse immunitaire induite contre G1A sous différentes formes,
- Figure 2 : Cinétique de la réponse immunitaire induite contre G1A présentée sous différentes formes,
- Figure 3 : Cinétique de la réponse immunitaire induite contre le porteur seul,
- Figure 4 : Stratégie de clonage par amplification génique de p40.

### Exemple 1 : Synthèse et Purification du G₁A

Le polypeptide de séquence 
Ser-Ile-Cys-Ser-Asn-Asn-Pro-Thr-Cys-Trp-Ala-Ile-Ser-Lys
|__________ S-S __________|
 noté G₁A, est préparé par synthèse en phase solide en utilisant la chimie Boc.

### Assemblage

L'assemblage du peptide est effectué par synthèse peptidique en phase solide sur polystyrène (divinylbenzène 1%), en commençant avec un agent de liaison Boc-Lys(2-cl-Z)-phénylacétamidométhyl.

On a utilisé la stratégie chimique Boc-Benzyle avec la procédure de déprotection-couplage suivante :

| | | |
|---|---|---|
| 1. | 55 % TFA dans DCM | (1 x 5 min) |
| 2. | 55 % TFA dans DCM | (1 x 25 min) |
| 3. | DCM | (2 x 1 min) |
| 4. | lsopropylalcool | (1 x 1 min) |
| 5. | DMF | (2 x 1 min) |
| 6. | 10% DIEA en DMF | (2 x 2 min) |
| 7. | Couplage | |
| 8. | DMF | (2 x 1 min) |
| 9. | DCM | (2 x 1 min) |

A chaque étape on utilise 20 ml de solvant par gramme de peptide-résine.

Le couplage est effectué dans du DMF avec un ester hydroxybenzotriazole préformé pendant 30 min. On vérifie à chaque étape du couplage, si des fonctions aminé libres résiduelles sont présentes, par le test à la ninhydrine. Si nécessaire, un double couplage est effectué.

Pour la synthèse du peptide G₁A, on a utilisé les groupes de protection de la chaîne latérale suivants :
- 2-chlorobenzyloxycarbonyl pour la Lysine,
- Benzyl pour la Sérine et la Thréonine,
- 4-méthylbenzyl pour la Cystéine,
- Formyl pour le Tryptophane.
   Avant l'étape finale de déprotection/cleavage, le groupe formyl est éliminé par traitement 30 min par une solution de piperidine à 25 % dans du DMF. La résine peptidique est lavée par du DCM et de l'éther, et séchée sous pression réduite.

### Clivage

Le peptide est clivé de la résine et complètement déprotégé par un traitement au Fluorure d'Hydrogène liquide. 10 ml de Fluorure d'Hydrogène par gramme de peptide-résine sont utilisés classiquement à 0°C pendant 45 min en présence de p-cresol et d'éthanedithiol comme piège. Après évaporation du Fluorure d'Hydrogène, le mélange de réaction brut est lavé à l'éther, dissout dans du TFA, précipité à l'éther et séché.

### Cyclisation et purification

Conditions générales de purification par HPLC :
- Phase stationnaire :: silice en C₁₈, 15-25 µm, 100 Å
- Phase mobile :: solvant A : eau 0,1 % TFA
solvant B : acétonitrile/A, 60/40% (v/v)
- Gradient linéaire :: 20 à 50 % B en 30 min (première étape de purification)
15 à 40 % B en 30 min (seconde étape de purification)
- Vitesse du flux :: 40 ml/min
- Détection :: UV (210 nm)

Le peptide brut obtenu après clivage est purifié dans les conditions décrites ci-dessus (gradient de 20 à 50 % B). Les fractions ayant une pureté supérieure à 70-80 % (HPLC) sont réunies et lyophilisées. Le peptide est ensuite purifié dans un mélange acétonitrile eau et DMSO (1mg/ml) et laissé sous agitation jusqu'à ce que la cyclisation soit complète (4 à 6 jours). L'évolution de la réaction est contrôlée par HPLC. Le mélange de réaction est finalement concentré sur la colonne d'HPLC préparative et un gradient de 15 à 40 96 de B est appliqué en 30 min afin de purifier le peptide.

Généralement, après lyophilisation, une seconde purification dans les mêmes conditions, est effectuée pour atteindre le degré de pureté requis.

La pureté et l'identité du produit final sont contrôlées par HPLC analytique, analyse des amino acides et analyse de masse FAB.

Dans le peptide ainsi obtenu, le résidu sérine en treizième position remplace le résidu Cys du peptide naturel, évitant ainsi une hétérogénéité dans la formation des ponts disulfures, pouvant être nuisible à l'immunogénicité.

### Exemple 2 : Préparation de l'épitope G₂AδCys

### Construction de gène : matériels et méthodes

Dans un microtube Eppendorf, on rince 300 µg de billes avec du tampon washing/binding (1M NaCl, 10mM Tris-HCl pH7,5, 1 mM EDTA) avant d'ajouter 0,2 pmole de l'oligo biotinylé, 15 minutes d'incubation à température ambiante pour le binding. Les billes avec l'oligo fixé sont rincées et sédimentées. 0,2 pmole de l'oligo phosphorylé en 5' suivant est ajouté dans 60 µl de tampon hybridation/ligation (50mM Tris-HCl pH7,6, 10 mM MgCl₂, 1 mM ATP, 1mM 1,4-dithiothreitol [DTT], 5% polyéthylène glycol [PEG] 8000). Le mélange d'hybridation est incubé à 70° C pendant 5 mn et laissé revenir à 37° C avant d'ajouter 3 unités de T4 DNA ligase (BRL) suivi de 15 mn d'incubation à 37° C. Le mélange réactionnel est rincé avant d'ajouter 0,2 pmole d'oligo suivant. La procédure d'hybridation/ligation est répétée autant de fois qu'on ajoute un nouveau oligo complémentaire phosphorylé en 5'. A la fin, le duplex d'ADN fixé sur billes magnétiques peut être séparé du support en coupant avec les enzymes de restriction appropriées.

On prépare l'ADN correspondant à la séquence G2AδCys et à la séquence G2AδCys liée à la protéine de liaison à la serumalbumine humaine(BB) notée BB-G2AδCys.

La séquence nucléotidique est exprimée chez E. coli pour récupérer les protéines correspondantes.

### Vecteur d'expression :

pVABBG2AδC est un vecteur d'expression de type intracellulaire, il contient un promoteur d'origine *E coli ,* l'opération tryptophane (Trp), suvi du gène codant pour le récepteur de la sérum albumine humaine BB (P-A Nygrén et col, J. Mol. Recognit., 1988, 1, 60) et enfin le gène codant pour G2AδC du VRS. L'expression du gène hétérologue peut être induite en présence de l'IAA (acide-3-β-indolacrylique). Le produit de fusion BBG2AδC peut être purifié par affinité sur colonne HSA-sépharose, après avoir libéré les protéines cytoplasmiques de *E coli.*

### Exemples de purification de protéines à partir de culture de 500 ml :

La souche *E. coli* RV 308 (Maurer et col., J. Mol. Biol., 1980, 139, 147) transfectée par le plasmide pVABBG2AδC, a été sélectionnée sur gélose renfermant de l'ampicilline (100 µg/ml) et de la tétracycline (8 µg/ml). On inocule la souche dans un Erlenmeyer contenant 100 ml de milieu de culture TSB (Tryptic Soy broth, Difco) (30g/l), supplémenté avec de la levure (Yeast Extract, Difco) (5 g/l), de l'Ampicilline (100 µg/ml), de la tétracycline (8 µg/ml) et du Tryptophane (100 µg/ml). Incuber à 32°C pendant 12 heures sous agitation (190 rpm). Transvaser la culture dans un autre erlenmeyer (5 litres) contenant quatre fois le volume initial (400 ml TSB + levure + les mêmes antibiotiques à la même concentration). Lorsque la densité optique du milieu (à 550 nm) atteint environ une D.O. de 1,5, on induit la production des protéines en ajoutant dans le milieu de l'IAA à la concentration finale de 25 µg/ml. On arrête la culture après 5 heures d'incubation, sous agitation (190 rpm) à 32°C. Après centrifugation, le culot bactérien est resuspendu dans un récipient contenant environ 60 ml de solution de TST (50 mM TrisHCl, pH 8,0, 200mM NaCl, 0,05 % Tween 20, 0,5 mM EDTA) à froid.

On introduit dans le récipient une sonde standard de sonicateur (VIBRA-CELL, Somics Mat, USA). On fait la sonication à la puissance 5 pendant deux minutes environ. Le surnageant de solution après centrifugation est filtré à 0,45 µm, et passé dans une colonne contenant environ 3 ml de gel de HSA-sépharose (STAHL et col, J. Immunol. Meth.,1989, 124. 43).

Les protéines purifiées sont analysées par SDS-PAGE sur l'appareil Phast System (PHARMACIA) ou sur Mini Protean BIORAD. Les gels sont révélés par le bleu de Coomassie. La protéine BBG2AδC, représentant plus de 90 % de pureté, correspond bien à la taille attendue (39,3 Kda) par rapport aux standards de poids moléculaires connus.

L'immunotransfert de cette protéine sur membrane Problott (ABI) permet de l'identifier avec des anticorps spécifiques, anti-BB et/ou anti-protéine G du VRS (ss-groupe A). Le rendement de protéines solubles purifiées à partir du cytoplasme de *E. coli* est environ 50 mg/litre de culture.

En fermenteur de 2 litres, on peut obtenir de 500 à 800 mg de protéines BBG2AδC par litre de culture, dans les conditions optimales de culture.

### Exemple 3 : isolement et purification de la protéine p40 naturelle

Le procédé de purification de la protéine P40 à partir de la biomasse de Klebsiella pneumoniae, souche I-145, a été mis au point avec un objectif principal : mettre au point un procédé permettant la transposition àgrande échelle et l'extrapolation industrielle. Ce procédé met en jeu successivement la préparation d'une fraction enrichie en protéines membranaires et la purification de la protéine P40 par chromatographie.

### MATERIEL ET METHODES

La biomasse de Klebsiella pneumoniae (souche 1-145, 40 g de cellules sèches) est ajustée à pH 2,5 à l'aide d'acide acétique pur.
Après addition de 1/2 volume d'une solution contenant 6 % cétrimide, 60 % éthanol, 1,5 M CaC12 dont le pH est ajusté à 2,5 avec de l'acide acétique, le mélange est placé sous agitation pendant 16 heures à température ambiante.

Après centrifugation 20 mn à 15000 g à 4°C, les protéines du surnageant sont précipitées à l'éthanol. Deux précipitations successives avec centrifugation intermédiaire (10 mn, 10000 g, 4 °C) sont réalisées : de 20 à 50 % puis de 50 à 80 %.

Les culots obtenus après la seconde précipitation sont remis en suspension dans une solution de zwittergent 3-14, 1 %.

Après agitation 4 heures à température ambiante, le pH est ajusté à 6,5 à l'aide de NaOH1 N.

Une centrifugation du mélange pendant 20 mn à 10000 g à 4 °C permet d'obtenir une fraction enrichie en protéines membranaires (fraction MP).

Les protéines de la fraction MP sont dialysées contre un tampon Tris/HCl 20 mM pH 8,0 ; zwittergent 3-14, 0,1 %. Le dialysat est déposé sur une colonne contenant un support de type échangeur d'anions forts (colonne de diamètre = 50 mm x H = 250 mm, gel Biorad Macroprep High Q) équilibrée dans le tampon décrit ci-dessus. La protéine P40 est éluée pour une concentration de 50 mM en NaCl dans le tampon d'équilibration.

Les fractions contenant la P40 sont rassemblées et dialysées contre un tampon citrate 20 mM pH 3,0 ; zwittergent 3-14, 0,1 %. Le dialysat est déposé sur une colonne contenant un support de type échangeur de cations forts (dimensions de la colonne : diamètre = 25 mm x H = 160 mm, gel Biorad Macroprep High S) équilibrée dans le tampon citrate 20 mM pH 3,0, zwittergent 3-14, 0,1 %. La protéine P40 est éluée pour une concentration 0,7 M en NaCl. Les fractions contenant la P40 sont rassemblées et concentrées par ultrafiltration à l'aide d'un système de filtration à flux tangentiel Minitan Millipore utilisé avec des plaques de membranes possédant un seuil de coupure 10 kDa.

### RESULTATS

Les fractions obtenues après chaque étape chromatographique sont analysées par SDS-PAGE afin de rassembler celles contenant la protéine P40.

Les quantités de protéines sont mesurées par la méthode de Lowry (tableau I). La pureté et l'homogénéité de la protéine P40 sont estimées par SDS-PAGE, en présence de standards de masse moléculaire.

Après l'étape de chromatographie d'échange de cations, la protéine P40 est dépourvue du contaminant majeur présent dans la fraction MP (la protéine présentant une masse moléculaire apparente de 18 kDa) et présente un degré de pureté supérieur à 95 %.

Le profil électrophorétique de la P40 révèle plusieurs bandes. Ces bandes sont reconnues après immunoblot par des anticorps monoclonaux P40 obtenus chez la souris. La bande majeure supérieure correspond à la protéine dénaturée (par le traitement à 100°C, 15 min. en présence,de SDS), et la bande mineure inférieure à la protéine sous sa forme native.

La P40 est en effet une protéine dite "heat-modifiable", et nous avons pu vérifier cette propriété à l'aide d'une cinétique de chauffage à 100°C en présence de SDS. Sans chauffage la protéine sous forme native présente une structure en hélices α qui fixe plus de SDS et migre donc plus loin vers l'anode que la forme dénaturée (dénaturation complète après 5 min. à 100°C) qui présente une structure en feuillets β (K.B KELLER (1978) J. Bacteriol. 134, 1181-1183).

La contamination par les lipopolysaccharides (LPS) est estimée par dosage par chromatographie en phase gazeuse de l'acide β-hydroxymyristique, acide gras marqueur des LPS de Klebsiella pneumoniae (tableau 1).

**Tableau 1 : Tableau récapitulatif des quantités de protéine et LPS des fractions obtenues pour les différentes étapes du procédé de purification de la protéine p40 (n.d. = non déterminé).**

| | PROTEINES | RENDEMENT | LPS |
|---|---|---|---|
| BIOMASSE | 40 g | - | n.d. |
| FRACTION MP | 900 mg | 2,25 % | n.d. |
| FRACTION ENRICHIE EN P40 | 400 mg | 1% | 10 % |
| PROTEINE P40 | 130 mg | 0,3 % | < 1% |

Cette méthode est utilisée pour approcher la teneur en LPS des échantillons issus des différentes étapes de purification.

La quantité d'acide β-hydroxymyristique présente dans la fraction P40 après chromatographie d'échange de cations étant inférieure au seuil de quantification du dosage, on peut estimer que la quantité de LPS résiduel est inférieure à 1 %.

### Exemple 4 : clonage de la protéine p40 et expression de BBp40

### SOUCHES BACTERIENNES

* E coli : RV 308 : souche ATCC 31608 (MAURER R., MEYER B.J., PTASCHNE M., J. MOL BIOL, 1980, 139,147-161).
* K. pneumoniae : IP 145 : souche C.I.B.P.F -

### VECTEURS

* pRIT 28 (Hultman et Col, 1988,7 : 629-638) : vecteur de clonage et de séquençage possédant le gène de résistance à l'ampicilline, les origines de réplication d'Ecoli et du phage F1 ainsi qu'une portion du gène lac-z d'E.coli (β-galactosidose).
* pVABB : vecteur d'expression de fusion de gène.

### SOLUTIONS

* Amplification génique :

| | |
|---|---|
| Tampon de lyse : | 25 mM Taps pH 9.3 |
| | 2 mM MgCl2 |
| Tampon d'amplification: | 25 mM Taps pH 9.3 |
| | 2 mM MgC12 |
| | tween 20 0.1 % |
| | 200 mM dNTP. |

* Purification des protéines :

| | | | |
|---|---|---|---|
| TST (20X) : | Tris base | 0,5 M | |
| | HCl | 0,3 M | |
| | NaCl | 4 M | |
| | Tween 20 | 1 % | |
| | EDTA | 20 mM | |
| Tampon de lavage : | Tris HCl | 50 mM | pH 8,5 |
| | MgC12 | 5 mM | |
| Solution de dénaturation : | Gua-HCl | 7,8 M | |
| | Tris-HCl | 28 mM | pH 8,5 |
| Solution de renaturation : | Gua-HCl | 0,5 M | |
| | Tris-HCl | 25 mM | pH 8,5 |
| | NaCl | 150 mM | |
| | Tween 20 | 0,05 96. | |

### MATERIEL ET METHODE

### - Synthèse des oligonucléotides

Les amorces nucléotidiques ont été déterminées à partir de la partie de la séquence publiée de l'OMPA de Klebsiella pneumoniae (LAWRENCE, G.J., et al, Journal of général microbiology, 1991, 137, 1911-1921) de la séquence conscensus issue de l'alignement des séquences de 5 OMPA d'entérobactéries (E.coli, S.tryphimurium, S.marcescens, S.dysenteriae, E.aeroginosae), ainsi que des séquences de peptides obtenus par séquençage manuel.

Les oligonucléotides ont été synthétisés selon la méthode chimique des phosphoramidites sur l'appareil "Gene Assembler Plus" de Pharmacia.

### - Amplification génique par PCR du gène de P40

L'ADN de l'OMPA de Klebsiella pneumoniae a été amplifié de la manière suivante.

Une colonie de Klebsiella pneumoniae est lysée dans 10 µl de tampon de lyse par chauffage à 95 °C pendant 5 minutes.

1 µl de cette solution sert de source d'ADN pour les réactions d'amplification.

Celles-ci sont réalisées dans 100 µl de tampon d'amplification (cf.annexe), avec 5 pmoles de chaque amorce et une unité d'enzyme Taq polymérase (Perkin Elmer Cetus). Chaque cycle comprend une étape de dénaturation de 30 secondes à 95°C suivie d'une hybridation de l'amorce à l'ADN et d'une extension d'une minute à 72 °C. 30 cycles sont ainsi effectués à l'aide d'un thermocycleur "Gen Amp PCR" 9000 Perkin Elmer Cetus .

Les PCR suivantes sont réalisées à partir des fragments d'ADN amplifiés précédemment.

Les fragments d'ADN amplifiés sont ensuite digérés, purifiés et liés au vecteur pRIT 28.

### SEQUENCAGE

Les fragments ainsi clonés sont séquencés sur un séquenceur automatique 373 DNA Séquenceur d'Applied Biosystem. Les réactions de séquençage sont réalisées à l'aide du kit "dye Terminator" selon les recommandations du fournisseur (Applied Biosystem) soit sur de l'ADN double brin obtenu après amplification génique ou issu de maxiprep soit sur de l'ADN simple brin issu de fragments PCR dénaturés (Hultman et Col, Nucleid acids res. ; 1989, 17 : 4937-4946).

### EXPRESSION DE LA PROTEINE

Le gène entier de P40 est cloné dans le vecteur d'expression pVABB. Ce vecteur permet d'adjoindre une queue d'affinité "BB" à P40 ; B étant la partie de la protéine G du streptocoque qui lie la serum albumine (Nygren P.A et Col ; Journal mol. Recognit. 1988 ; 1, 69-74).

Les souches d'Ecoli RV308 transformées par le vecteur pVABBP40 sont mises à cultiver une nuit à 37°C sous agitation, dans 100 ml de TSB complémenté en extrait de levure, en ampicilline (200 µg/ml) en tétracycline (8 µg/ml) et en tryptophane (100 µg/ml). Le lendemain, une culture à DO= 1 pour une longueur d'onde de 580 nm est préparée dans du TSB + extraits de levure + ampi + tetra.

Après 10 minutes de culture, l'expression de la protéine est induite par addition d'IAA à (25 µg/ml) dans le milieu . La culture est centrifugée à 4°C à 2460 g pendant 10 minutes.

Le culot est repris par 20ml de TST 1 x pH 7,4, et la solution est alors centrifugée à 4°C à 23000 g pendant 30 minutes.

Le surnageant est passé sur Sépharose ce qui permet d'isoler les protéines dites solubles. Le culot est lavé avec du tampon de lavage puis centrifugé à 23000 g à 4°C pendant 30 minutes. Le culot renfermant les corps d'inclusion est alors repris par 900 µl d'une solution dénaturante + 100 µl de Diothiothreitol 10mM et incubé 2 heures à 37 °C.

La solution est ensuite incubée 1 nuit à température ambiante, sous agitation,dans 100 ml de tampon de renaturation à 2300 g pendant 1 heure.

Le surnageant est passé sur HSA Sépharose.

Dans les deux cas les protéines fixées sont éluées avec de l'acide acétique 0,5 M pH 2,8 et collectées par fraction de 1 ml.

Les fractions collectées sont ensuite annalysées sur gel d'électrophorèse en SDS-PAGE et par Immuno blot.

### RESULTATS

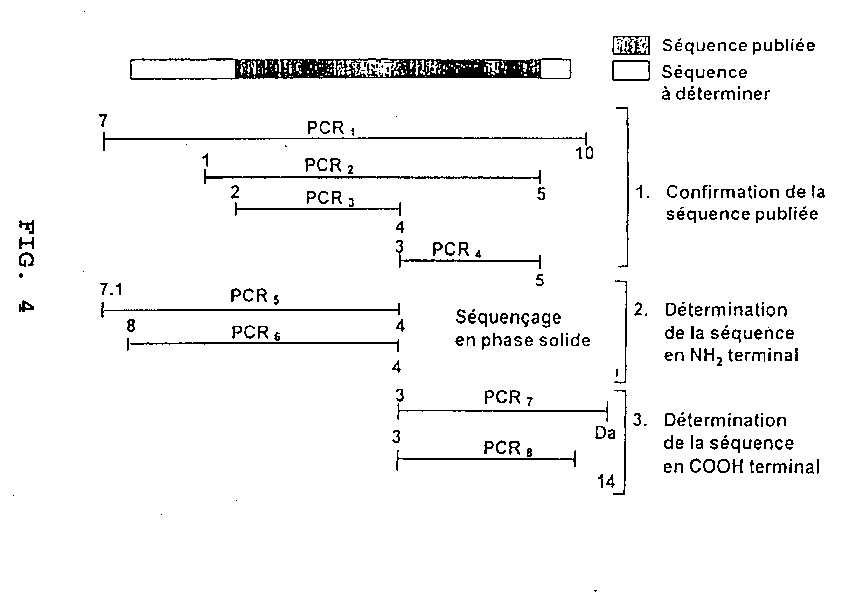

Le clonage du gène a été effectué en trois temps selon la stratégie présentée sur la figure 4.

Dans un premier temps, nous avons confirmé la partie de la séquence publiée à l'exception d'un T à la place d'un A en position 103.

Puis nous avons déterminé la séquence en 3 ' du gène et enfin celle en 5 '.

Le gène entier a été obtenu par fusion des deux parties 8/4 et 3/14 puis cloné dans le vecteur pRIT 28. La séquence correspond à SEQ ID N° 13.

La protéine est exprimée sous la forme BBP40.

Elle est essentiellement obtenue à partir des corps d'inclusion. Pour une culture de 200 ml, on purifie une quinzaine de milligrammes de protéine.

Le profil électrophorétique montre que BBP40, obtenue après dénaturation, est d'une grande pureté. Le poids moléculaire apparent, correspond au poids théorique calculé qui est de 63 kDa.

La caractérisation en Immuno blot montre que la protéine purifiée est bien reconnue par un sérum de lapin anti-P40.

### Exemple 5 : couplage de la protéine p40 au peptide G₁A

p40 (5 mg/ml, 40 mg) est dialysée contre 300 volumes de tampon phosphate de sodium 0,1 M pH 7, zwittergent 3-14, 0,1%.

Le dialysat est ajusté à une concentration de 2 mg/ml à l'aide d'un tampon carbonate 0,1 M pH 9 ; zwittergent 3-14, 0,1%. Du sodium dodécyl sulfate (SDS) est ajouté pour atteindre une concentration finale de 4%.

Le peptide G₁ (10 mg/10 ml de tampon carbonate 0,1 M pH 9 ; zwittergent 3-14 0,1 %) est ajouté à la solution de p40. La valeur du pH est contrôlée (comprise entre pH 9 et pH 10).

Ajouter 220 µl de glutaraldéhyde (2,5% dans l'eau), agiter 24 heures à 4° C.

Ajouter 5 ml de tampon carbonate 0,1 M pH 9 ; zwittergent 3-14 0,1%; vérifier le pH (compris entre pH 9 et pH 10) ; agiter 72 heures à 4° C.

Ajouter 220 µl de glutaraldéhyde (2,5% dans l'eau), vérifier le pH, agiter 24 heures à + 4° C.

La réaction est stoppée par addition de 100 µl de lysine 1 M. La solution est dialysée 24 heures à 4° C.

Le SDS est éliminé par double précipitation au KCl.

La solution contenant le conjugué p40 est congelée et utilisée telle quelle ou lyophylisée.

### Exemple 6 : activité

### Matériel et méthodes

Les souris C57BL/6 (N=5) sont immunisées à JO, J10, J20 par voie sous cutanée avec 10 µg de G1, couplé ou non à un porteur, en présence ou non d'un adjuvant. Le sérum est collecté et testé par EL1SA. Les Ig anti-G1 ou anti-porteur sont isolées sur support BSA-G1 et sur support "porteur" (KLH ou TT ou P40). Les Ig sont révélées à l'aide d'un conjugué anti-Ig lapin péroxydase. La densité optique est lue à 450 nm et le titre en anticorps anti-G1 est donné par l'inverse de la dernière dilution donnant deux fois le bruit de fond. Les résultats représentent la moyenne ± écart-type des titres des 5 souris.

### RESULTATS

### Induction d'une réponse immunitaire contre G1A

Les souris sont immunisées avec G1A sous différentes formes selon un schéma d'immunisation identique. Les réponses anticorps induites par les différentes formes de G1A sont comparées 28 jours après le début de l'expérience.

Le peptide synthétique G1A administré pur n'induit pas de réponse immunitaire même s'il est coadministré avec l'adjuvant de Freund. Présenté par le porteur KLH, G1A induit une réponse faible qui est significativement augmentée par la coadministration de l'adjuvant de Freund (AF). Présenté par p40, G1A induit une réponse supérieure à celle obtenue dans le schéma d'immunisation classique KLH/Gl+AF, p40 à des propriétés de "self-adjuvant carrier".

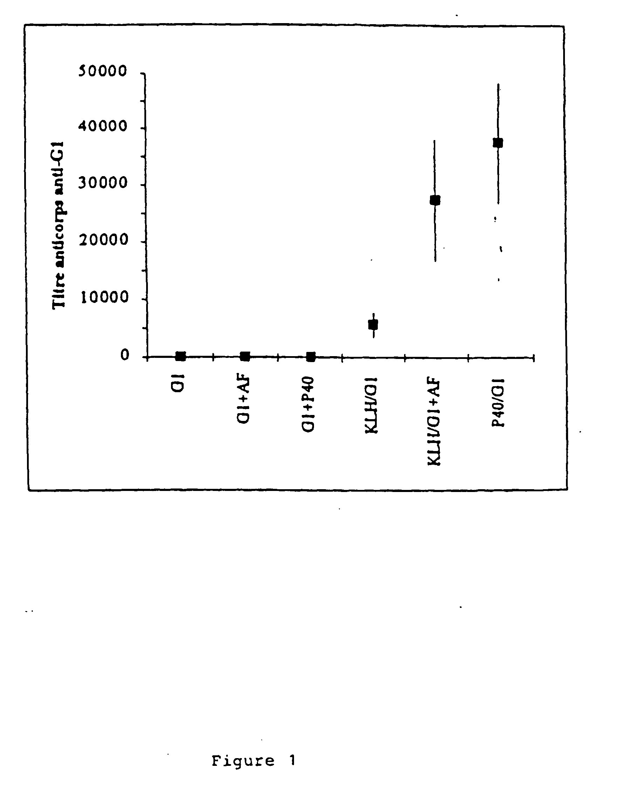

Les résultats sont présentés sur la figure 1.

### Cinétique de la réponse immunitaire contre G1A

Les souris sont immunisées avec G1A sous différentes formes selon un schéma d'immunisation identique. Les réponses anticorps induites par les différentes formes de G1A sont comparées dans le temps : 7, 17, 28, 35, 42 jours après le début de l'expérience.

La réponse anti-G1A est significativement plus élevée et plus rapide lorsque les souris sont immunisées avec p40/G1A que les immunisations plus classiques TT/G1A et KLH/G1A+AF. Une seule injection de p40/G1A permet d'obtenir, en 7 jours, un titre d'anticorps anti-G1A de 1000. Ce titre est obtenu avec TT/G1A ou KLH/G1A+AF en 28 jours. La réponse maximum (titre = 1/380 000), obtenue après trois injections, en 28 jours, est environ 30 fois supérieure à celle obtenue avec KLH/G1A+AF et 70 fois supérieure à celle obtenue avec TT/G1A. Le titre en anticorps anti-G1A se maintient sans faiblir jusqu'au jour 42.

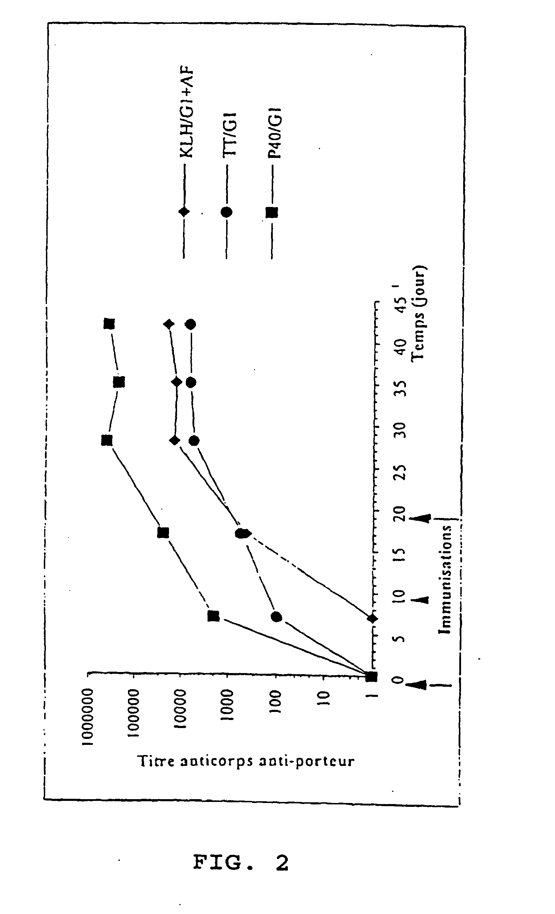

Les résultats sont présentés sur la figure 2.

### Cinétique de la réponse immunitaire contre le porteur

Les souris sont immunisées avec G1A couplé à un porteur selon un schéma d'immunisation identique. Les réponses anticorps induites par lesdifférents porteurs sont comparées dans le temps, 7, 17, 28, 35, 42 jours après le début de l'expérience.

La réponse anti-p40 (titre voisin du 10 000) est supérieure à la réponse anti-KLH mais non significativement différente de la réponse anti-TT.

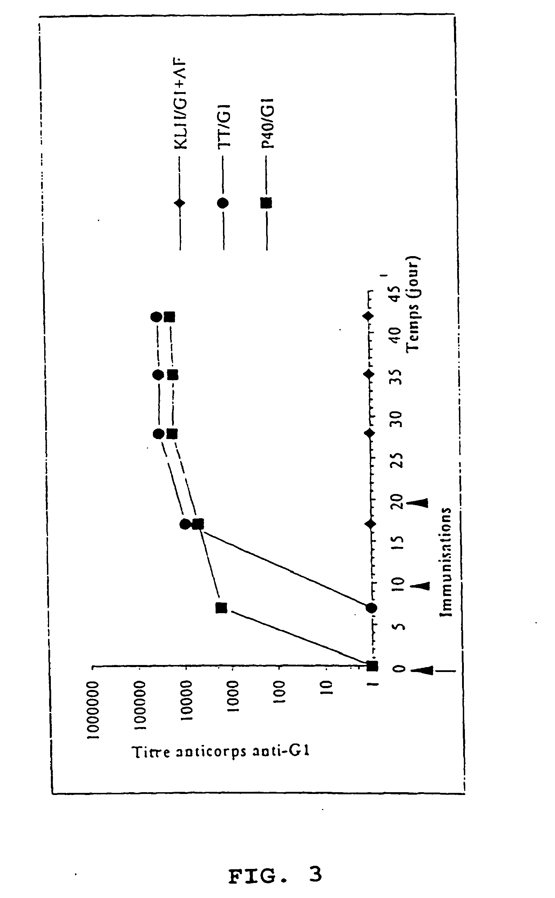

Les résultats sont présentés sur la figure 3.

### CONCLUSION

Le couplage chimique du peptide G1A sur la protéine p40 a permis d'induire une réponse anti-G1A significativement plus importante et plus rapide que celles provoquées par les modèles de référence KLH/G1A+AF ou TT/G1A. Le couplage du peptide G1B devrait induire des réponses similaires.

### Exemple 7 : Evaluation du potentiel protecteur des peptides et des protéines recombinantes de la glycoprotéine G du virus respiratoire syncytial (VRS) sous- groupe A couplés à la protéine porteuse p40

Les souris BALB/c ont été immunisées avec les différentes préparations suivantes :
1) peptide de synthèse G1A couplé à KLH (keyhole limpet hemocyanin) = KLH.G1A.
2) peptide de synthèse G1A couplé à la protéine porteuse p40 = p40.GlA.
3) témoin p40 seul.
4) protéine recombinante produite dans E. coli : BBG2AδC couplée à la protéine porteuse p40 = p4O.BBG2AδC.
5) peptide de synthèse G1A couplé à la protéine porteuse toxine tétanique (TT) = TT.G1A.
6) témoin TT seul.
7) témoin BB seul.
8) témoin VRS long (sous-groupe A).

Les souris ont reçu 3 doses intramusculaires (200 µg/souris) avec l'hydroxyde d'aluminium comme adjuvant (utilisé couramment chez l'homme). Les résultats des tests de protection ainsi que du profil immunologique des sérums se trouvent dans le tableau 2.

Les préparations suivantes confèrent une protection complète suite au challenge avec le VRS Long (Souche A) : p40.G1A, p40.BBG2AδC, par rapport à TT.G1A qui confère aussi une très bonne protection comparable au peptide KLH.G1A. En test ELISA, tous reconnaissent l'antigène VRS avec un titre le plus fort pour p40.G1A=1/12800.

Quant au test de neutralisation, aucune des préparations ne possède d'activité neutralisante in vitro.

**Tableau 2 : Protection conférée et profil immunologique des sérums après challenge avec VRS Long (A) suite à l'immunisation de souris BALB/c avec différentes protéines recombinantes. (3-4 semaines après 3 doses i.m. avec hydroxide d'Aluminium)**

| Peptides et Protéines Recombinantes | Protection | | | | Titre Elisa versus VRS long | Neutralisation log 2/25 µl |
|---|---|---|---|---|---|---|
| | DICT₅₀ log10/g poumons challenge avec VRS long (1.5.10⁵/souris) (Sous-Groupe A) | | | | | |
| | 5 - 6 jours | | 7 - 8 jours | | | |
| | 2,45 | | 2,45 | | | |
| KLH._{G1A} | 2,45 | ≤ 2,0 ± 0,4 | 2,15 | ≤ 2,0 ± 0,4 | | |
| (100 à 157 µg) | < 1,7 | p< 0,001 | < 1,7 | p< 0,001 | 4000 | < 3,0 |
| | < 1,7 | | < 1,7 | | | |
| | < 1,7 | | < 1,7 | | | |
| | < 1,7 | | < 1,7 | | | |
| P40. G1A | < 1,7 | < 1,7 ± 0 | < 1,7 | < 1,7 ± 0 | 12 800 | < 3,0 |
| (200 µg) | < 1,7 | p<0,001 | < 1,7 | p< 0,001 | | |
| | < 1,7 | | < 1,7 | | | |
| | 4,7 | | 4,7 | | | |
| Témoins P40 | 4,45 | 4,5 ± 0,1 | 4,45 | 4,5 ± 0,1 | 300 | <3,0 |
| (200 µg) | 4,45 | p< 0,001 | 4,45 | p< 0,001 | | |
| | 4,45 | | 4,45 | | | |
| | <1,7 | | <1,7 | | | |
| P40. BBG2AδC | < 1,7 | < 1,7 ± 0 | < 1,7 | < 1,7 ± 0 | | |
| (200 µg) | < 1,7 | p<0,001 | < 1,7 | p<0,001 | 1700 | <3,0 |
| | <1,7 | | <1,7 | | | |
| | <1,7 | | <1,7 | | | |
| | <1,7 | | <1,7 | | | |
| TT.G1A | <1,7 | < 1,9 ± 0,3 | <1,7 | <1,9 ± 0,3 | | |
| (200µg) | <1,7 | p<0,001 | < 1,7 | p < 0,001 | 7200 | <3,0 |
| | <1,7 | | <1,7 | | | |
| | 2,45 | | 2,45 | | | |
| | 4,45 | | 4,7 | | | |
| TT Témoins | 4,2 | 4,2 ± 0,3 | 4,2 | 4,2 ± 0,4 | | |
| (200 µg) | 4,2 | p=0.022 | 4,2 | p=0,053 | 250 | < 3,0 |
| | 4,45 | | 4,45 | | | |
| | 3,7 | | 3,7 | | | |
| | 2,95 | | 2,95 | | | |
| Témoins BB | 4,2 | 3,7 ± 0,5 | 4,2 | 3,8 ± 0,5 | 150 | < 3,0 |
| (200 µg) | 3,95 | p=0,853 | 4,2 | p=0,760 | | |
| | 3,7 | | 3,7 | | | |
| | 3,7 | | 3,7 | | | |
| | <1,7 | | <1,7 | | | |
| | < 1,7 | <1,7 ± 0 | < 1,7 | < 1,7 ± 0 | | |
| Témoins VRS long | <1,7 | p=0,001 | < 1,7 | p=0,001 | 76800 | 6,6 |
| | <1,7 | | <1,7 | | | |
| | <1,7 | | <1,7 | | | |
| | 3,95 | | 3,95 | | | |
| | 3,95 | | 4,2 | | | |
| Témoins, non immunisés, | 3,7 | 3,7 ± 0,2 | 3,7 | 3,8 ± 0,3 | 150 | < 3,0 |
| challengés | 3,45 | | 3,45 | | | |
| | 3,95 | | 3,95 | | | |
| | 3,45 | | 3,7 | | | |
| Témoins, non immunisés, non challengés | Pas de virus | | Pas de virus | | 150 | < 3,0 |

### Exemple 8

Evaluation du potentiel protecteur des peptides de la glycoprotéine G du virus respiratoire syncytial (VRS) sous-groupe A et sous-groupe B couplés à la KLH. Protection vis-à-vis d'un challenge réalisé avec les deux sous-groupes du VRS.

Les souris BALB/c ont été immunisées avec les différentes préparations suivantes :
1. peptide de synthèse C1A couplé à la KLH (keyhole limpet hemocyanin) = KLH-G1A
2. peptide de synthèse G1B couplé à la KLH (keyhole limpet hemocyanin) = KLH-G1B. Le peptide G1B correspond à la séquence G (174-187)δCys du sous-groupe B dont la séquence est : 
Ser-Ile-Cys-Gly-Asn-Asn-Gln-Leu-Cys-Lys-Ser-Ile-Ser-Lys
|____________s-s____________|
3. Témoin KLH
4. Témoin VRS long (sous-groupe A)
5. Témoin VRS 8/60 (sous-groupe B)

Les souris ont reçu 3 doses intramusculaires (200 µg/souris) avec l'adjuvant de Freund. Les résultats des tests de protection ainsi que du profil immunologique des sérums se trouvent dans le tableau 3.

La préparation KLH-G1A permet une protection complète vis-à-vis du VRS sous-groupe A mais pas vis-à-vis du VRS sous-groupe B. Par contre, la préparation KLH-G1B permet une protection complète vis-à-vis du VRS sous-groupe B mais pas vis-à-vis du VRS sous-groupe A. Le test ELISA reflète la même situation.

**Tableau 3 : Protection conférée et profil immunologique des sérums après challenge avec le RS long (sous-groupe A) ou avec le RS 8/60 (sous-groupe B) suite à l'immunisation de souris BALB/c avec les peptides G1A et G1B.**

| **Peptides couplés à la KLII** | **PROTECTION DICT**_{**10**} **log 10/g poumons** | | **Titre ELISA** | |
|---|---|---|---|---|
| | **Challenge** | **Challenge** | **Versus** | **Versus** |
| | **VRS long (sous-groupe A)** | **VRS 8/60 (sous-groupe B)** | **VRS long (A)** | **VKS 8/60 (B)** |
| | 1,5 x 10⁵/s (50/µl) | 0,6x10⁵/s (50/µl) | | |
| G1A | ≤ 1,8 ± 0,3 | 3,3 ± 0,5 | | |
| | n = 11 p < 0,001 | n =10 p = 0,237 | 29866 | 266 |
| G1B | 3,8 ± 0.8 | ≤ 2,1 ± 0,5 | | |
| | n=7 p=0,517 | n=8 p< 0,001 | <100 | 7200 |
| Témoin KLII | 3,7 ± 0,3 | 3.4±0.3 | | |
| | n=11 p=0,01 | n=10 p=0,6 | ≤200 | 133 |
| Témoin VRS (A) | ≤ 1,7±0 | ≤1,7±0 | | |
| | n=11 p<0,001 | n=11 p<0,001 | ≥ 68 266 | 51200 |
| Témoin VRS (B) | ≤1,7±0 | ≤ 1,7±0 | | |
| | n=10 p<0,001 | n =10 p<0,001 | ≥76800 | 68266 |

### Exemple 9: Application vétérinaire

Evaluation du potentiel protecteur de peptide G1vΔC dérivé de la protéine G de la souche bovine du Virus Respiratoire Syncytial (VRS) Lerch et al. 1990, J. Virol. 64:5559 couplé à la protéine porteuse KLH. 
174 . 187
Ser Thr Cys Glu Gly Asn Leu Ala Cys Leu Ser Leu Ser His
 présentant un pont disulfure en position 176-182.

Le peptide préparé par synthèse en phase solide en utilisant la chimie Boc est couplé au KLH en utilisant la glutaraldéhyde (Schaaper et al. Mol. Immunol. (1989) 26: 81-85).

Deux veaux ont été immunisés par voie intramusculaire avec 500 µg de G1vΔC-KLH avec de l'adjuvant incomplet de Freund 3 fois à intervalle de 3 semaines. Un veau a été immunisé avec KLH sans peptide G1VΔC; avec un adjuvant incomplet de Freund.

Les animaux sont challengés avec la souche Snook, 21 jours, après la dernière inoculation, par voie intranasale et intratrachéale avec chacune 1ml de virus titrant à 2x105 /ml.

Le virus titré sur cellules de reins de veau selon la méthode des plaques est déterminé dans les lavages nasopharyngeaux respectivement 3 et 2 jours après le challenge et 7 jours dans les poumons des animaux sacrifiés.

### REPONSE EN ANTICORPS CIRCULANTS :

Veau 3432 (KLH + FIA):

| **Date** | **Traitement** | **Titre log10 ELISA** | | | |
|---|---|---|---|---|---|
| | | **Peptide + KLH** | **Peptide** | **KLH** | **BRSV (Snook)** |
| 23/11 | J0 vaccination | < 1.0 | < 1.0 | < 1.0 | < 1.5 |
| 14/12 | J21 Vaccination | < 1.0 | < 1.0 | 3.0 | < 1.5 |
| 04/01 | J42 vaccination | < 1.0 | < 7.0 | 4.7 | < 1.5 |
| 01/02 | J70 VRS IN/ IT | <1.0 | <1.0 | 5.7 | < 1.5 |
| 08/02 | J77 sacrifice | 1.5 | < 1.0 | 4.8 | < 1.5 |

Veau 3440 (Peptide - KLH + FIA)

| **Date** | **Traitement** | **Titre log 10 ELISA** | | | |
|---|---|---|---|---|---|
| | | **Peptide + KLH** | **Peptide** | **KLH BRSV (Snook)** | |
| 23/11 | J0 vaccination | <1.0 | <1.0 | <1.0 | < 1.5 |
| 14/12 | J21 Vaccination | 1.6 | <1.0 | <1.0 | <1.5 |
| 04/01 | J42 vaccination | 3.8 | 2.6 | 1.7 | 1.9 |
| 01/02 | J70 VRS IN/IT | 2.7 | 2.8 | 2.6 | 3.7 |
| 08/02 | J77 sacrifice | 4.1 | 2.6 | 1.7 | 3.1 |

Veaux auxquels a été administré 500 µg G1vΔC - KLHen adjuvant incomplet de Freund à trois occasions à 3 semaines d'intervalles.

### REPONSE AU CHALLENGE DU VIRUS

| **Veaux** | **Vaccination** | **Relargage nasopharyngéal** | | **J7 Virus du poumon** | | **% pneumoniae** |
|---|---|---|---|---|---|---|
| | | **No jours** | **Titre max.** | **Titre LBA (pfu/ml)** | **Poumon Homog.** | |
| 3432 | KLH + FIA | 3 | 5.1 x 10³ | 1.4 x 10² | 3/3 | 12 |
| 3440 | Peptide - KLH + FIA | 2 | 5.5 x 10² | < 0.7 | 0/3 | < 1 |

### REPONSE EN ANTICORPS CIRCULANTS

| **Veaux** | **Vaccination** | **Titre log10 ELISA (BRSV Snook)** | | | | |
|---|---|---|---|---|---|---|
| | | **J0** | **J24** | **J42** | **J68** | **J75** |
| 4138 | KLH+FIA | < 1.5 | < 1.5 | <1.5 | <1.5 | 2.4 |
| 4140 | Peptide - KLH + FIA | < 1.5 | < 1.5 | 3.0 | 2.5 = | 2.9 |

| | | | | | | |
|---|---|---|---|---|---|---|
| *Veau auquel a été administré 500 µg de BP 4006 . KLH en adjuvant incomplet de Freund à trois occasions à trois semaines d'intervalle. | | | | | | |

### REPONSE AU CHALLENGE DU VIRUS

| **Veaux** | **Vaccination** | **Relargage nasopharyngéal** | | **J7 Virus du poumon** | | **% pneumoniac** |
|---|---|---|---|---|---|---|
| | | **No jours** | **Titre max.** | **Titre LBA** (pfu/ml) | **Poumon** Homog. | |
| 4138 | KLH + FIA | 5 | 4 x 10¹ | 6.5 x 10² | 2/3 | 27 |
| 4140 | Peptide - KLH + FIA | 4 | 2 x 10³ | 7.0 x 10¹ | 3/3 | 2 |

### LISTE DE SEQUENCES

(1) INFORMATIONS GENERALES:
   (i) DEPOSANT:
      (A) NOM: PIERRE FABRE MEDICAMENT
      (B) RUE: 17, AVENUE JEAN MOULIN
      (C) VILLE: CASTRES
      (E) PAYS: FRANCE
      (F) CODE POSTAL: 81106
   (ii) TITRE DE L' INVENTION: ELEMENT D'IMMUNOGENE, AGENT IMMUNOGENE, COMPOSITION PHARMACEUTIQUE ET PROCEDE DE PREPARATION.
   (iii) NOMBRE DE SEQUENCES: 75
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
   (vi) DONNEES DE LA DEMANDE ANTERIEURE:
      (A) NUMERO DE LA DEMANDE: FR 94 04009
      (B) DATE DE DEPOT: 06-AVRIL-1994

Information pour la SEQ ID NO : 1 G2A
   TYPE DE SEQUENCE : acides aminés et nucléotides
      LONGUEUR DE LA SEQUENCE : 101 acides aminés. 303 nucléotides
      NOMBRE DE BRINS : simple
      CONFIGURATION : linéaire
   TYPE DE MOLECULE : protéine 
130
N - Thr Val Lys Thr Lys Asn Thr Thr Thr Thr Gln Thr Gln Pro Ser Lys Pro Thr Thr Lys
5'- ACC GTG AAA ACC AAA AAC ACC ACG ACC ACC CAG ACC CAG CCG AGC AAA CCG ACC ACC AAA
150
Gln Arg Gln Asn Lys Pro Pro Asn Lys Pro Asn Asn Asp Phe His Phe Glu Val Phe Asn Phe
CAG CGT CAG AAC AAA CCG CCG AAC AAA CCG AAC AAC GAT TTC CAT TTC GAA GTG TTC AAC TTC
171 173 176 182 186
Val Pro Cys Ser Ile Cys Ser Asn Asn Pro Thr Cys Trp Ala Ile Cys Lys Arg Ile Pro Asn
GTG CCG TGC AGC ATC TGC AGC AAC AAC CCG ACC TGC TGG GCG ATC TGC AAA CGT ATC CCG AAC
192
Lys Lys Pro Gly Lys Lys Thr Thr Thr Lys Pro Thr Lys Lys Pro Thr Phe Lys Thr Thr Lys
AAA AAA CCG GGC AAA AAA ACC ACG ACC AAA CCG ACC AAA AAA CCG ACC TTC AAA ACC ACC AAA
213 230
Lys Asp His Lys Pro Gln Thr Thr Lys Pro Lys Glu Val Pro Thr Thr Lys Pro - C
AAA GAT CAT AAA CCG CAG ACC ACC AAA CCG AAA GAA GTG CCG ACC ACC AAA CCG - 3'
Information pour la SEQ ID N0 : 2 G2B
   TYPE DE SEQUENCE : acides aminés et nucléotides
      LONGUEUR DE LA SEQUENCE :101 acides aminés,303 nucléotides
      NOMBRE DE BRINS : simple
      CONFIGURATION : linéaire
   TYPE DE MOLECULE : protéine 
130
N - Thr Ala Gln Thr Lys Gly Arg Ile Thr Thr Ser Thr Gln Thr Asn Lys Pro Ser Thr Lys
5'- ACC GCG CAG ACC AAA GGC CGT ATC ACC ACC AGC ACC CAG ACC AAC AAA CCG AGC ACC AAA
150
Ser Arg Ser Lys Asn Pro Pro Lys Lys Pro Lys Asp Asp Tyr His Phe Glu Val Phe Asn Phe
AGC CGT AGC AAA AAC CCG CCG AAA AAA CCG AAA GAT GAT TAC CAC TTC GAA GTG TTC AAC TTC
171 173 176 182 186
Val Pro Cys Ser Ile Cys Gly Asn Asn Gln Leu Cys Lys Ser Ile Cys Lys Thr Ile Pro Ser
GTG CCC TGC AGC ATC TGC GGC AAC AAC CAG CTG TGC AAA AGC ATC TGC AAA ACC ATC CCG AGC
192
Asn Lys Pro Lys Lys Lys Pro Thr Ile Lys Pro Thr Asn Lys Pro Thr Thr Lys Thr Thr Asn
AAC AAA CCG AAA AAG AAA CCG ACC ATC AAA CCG ACC AAC AAA CCG ACC ACC AAA ACC ACC AAC
213 230
Lys Arg Asp Pro Lys Thr Pro Ala Lys Met Pro Lys Lys Glu Ile Ile Thr Asn - C
AAA CGT GAT CCG AAA ACC CCG GCG AAA ATG CCG AAG AAG GAA ATC ATC ACC AAC - 3'
Information pour la SEQ ID NO : 3 G2AδCys
   TYPE DE SEQUENCE: acides aminés et nucléotides
      LONGUEUR DE LA SEQUENCE : 101 acides aminés, 303 nucléotides
      NOMBRE DE BRINS : simple
      CONFIGURATION : linéaire
   TYPE DE MOLECULE : protéine 
130
N - Thr Val Lys Thr Lys Asn Thr Thr Thr Thr Gln Thr Gln Pro Ser Lys Pro Thr Thr Lys
5'- ACC GTG AAA ACC AAA AAC ACC ACG ACC ACC CAG ACC CAG CCG AGC AAA CCG ACC ACC AAA
 
150
Gln Arg Gln Asn Lys Pro Pro Asn Lys Pro Asn Asn Asp Phe His Phe Glu Val Phe Asn Phe
CAG CGT CAG AAC AAA CCG CCG AAC AAA CCG AAC AAC GAT TTC CAT TTC GAA GTG TTC AAC TTC
171 173 176 182 186
Val Pro Ser Ser Ile Cys Ser Asn Asn Pro Thr Cys Trp Ala Ile Ser Lys Arg Ile Pro Asn
GTG CCG AGC AGC ATC TGC AGC AAC AAC CCG ACC TGC TGG GCG ATC AGC AAA CGT ATC CCG AAC
192
Lys Lys Pro Gly Lys Lys Thr Thr Thr Lys Pro Thr Lys Lys Pro Thr Phe Lys Thr Thr Lys
AAA AAA CCG GGC AAA AAA ACC ACG ACC AAA CCG ACC AAA AAA CCG ACC TTC AAA ACC ACC AAA
213 230
Lys Asp His Lys Pro Gln Thr Thr Lys Pro Lys Glu Val Pro Thr Thr Lys Pro - C
AAA GAT CAT AAA CCG CAG ACC ACC AAA CCG AAA GAA GTG CCG ACC ACC AAA CCG - 3'
Information pour la SEQ ID NO : 4 G2BδCys
   TYPE DE SEQUENCE : acides aminés et nucléotides
      LONGUEUR DE LA SEQUENCE : 101 acides aminés, 303 nucléotides
      NOMBRE DE BRINS : simple
      CONFIGURATION : linéaire
   TYPE DE MOLECULE : protéine 
130
N - Thr Ala Gln Thr Lys Gly Arg Ile Thr Thr Ser Thr Gln Thr Asn Lys Pro Ser Thr Lys
5'- ACC GCG CAG ACC AAA GGC CGT ATC ACC ACC AGC ACC CAG ACC AAC AAA CCG AGC ACC AAA
150
Ser Arg Ser Lys Asn Pro Pro Lys Lys Pro Lys Asp Asp Tyr His Phe Glu Val Phe Asn Phe
AGC CGT AGC AAA AAC CCG CCG AAA AAA CCG AAA GAT GAT TAC CAC TTC GAA GTG TTC AAC TTC
171 173 176 182 186
Val Pro Ser Ser Ile Cys Gly Asn Asn Gln Leu Cys Lys Ser Ile Ser Lys Thr Ile Pro Ser
GTG CCC AGC AGC ATC TGC GGC AAC AAC CAG CTG TGC AAA AGC ATC AGC AAA ACC ATC CCG AGC
192
Asn Lys Pro Lys Lys Lys Pro Thr Ile Lys Pro Thr Asn Lys Pro Thr Thr Lys Thr Thr Asn
AAC AAA CCG AAA AAG AAA CCG ACC ATC AAA CCG ACC AAC AAA CCG ACC ACC AAA ACC ACC AAC
213 230
Lys Arg Asp Pro Lys Thr Pro Ala Lys Met Pro Lys Lys Glu Ile Ile Thr Asn - C
AAA CGT GAT CCG AAA ACC CCG GCG AAA ATG CCG AAG AAG GAA ATC ATC ACC AAC - 3'
Information pour la SEQ ID NO :5 5 G1ACys
   TYPE DE SEQUENCE : acides aminés et nucléotides
      LONGUEUR DE LA SEQUENCE : 14 acides aminés, 42 nucléotides
      NOMBRE DE BRINS : simple
      CONFIGURATION : linéaire
   TYPE DE MOLECULE : peptide 
174 176 182 186 187
N - Ser Ile Cys Ser Asn Asn Pro Thr Cys Trp Ala Ile Cys Lys - C
5'- AGC ATC TGC AGC AAC AAC CCG ACC TGC TGG GCG ATC TGC AAA - 3'
Information pour la SEQ ID NO : 6 GIBCys
   TYPE DE SEQUENCE: acides aminés et nucléotides
      LONGUEUR : DE LA SEQUENCE : 14 acides aminés, 42 nucléotides
      NOMBRE DE BRINS : simple
      CONFIGURATION : linéaire
   TYPE DE MOLECULE : peptide 
174 176 182 186 187
N - Ser Ile Cys Gly Asn Asn Gln Leu Cys Lys Ser Ile Cys Lys - C
5'- AGC ATC TGC GGC AAC AAC CAG CTG TGC AAA AGC ATC TGC AAA - 3'
Information pour la SEQ ID NO : 7 G1A
   TYPE DE SEQUENCE : acides aminés et nucléotides
      LONGUEUR DE LA SEQUENCE: 14 acides aminés, 42 nucléotides
      NOMBRE DE BRINS : simple
      CONFIGURATION : linéaire
   TYPE DE MOLECULE : peptide 
174 176 182 186 187
N - Ser Ile Cys Ser Asn Asn Pro Thr Cys Trp Ala Ile Ser Lys - C
5'- AGC ATC TGC AGC AAC AAC CCG ACC TGC TGG GCG ATC AGC AAA - 3'
Information pour la SEQ ID NO : 8 G1B
   TYPE DE SEQUENCE : acides aminés et nucléotides
      LONGUEUR DE LA SEQUENCE : 14 acides aminés, 42 nucléotides
      NOMBRE DE BRINS : simple
      CONFIGURATION : linéaire
   TYPE DE MOLECULE : peptide 
174 176 182 186 187
N - Ser Ile Cys Gly Asn Asn Gln Leu Cys Lys Ser Ile Ser Lys - C
5'- AGC ATC TGC GGC AAC AAC CAG CTG TGC AAA AGC ATC AGC AAA - 3'
Information pour la SEQ ID NO: 9 GL'A
   TYPE DE SEQUENCE : acides aminés
      LONGUEUR DE LA SEQUENCE : 14 acides aminés
      NOMBRE DE BRINS : simple
      CONFIGURATION : linéaire
   TYPE DE MOLECULE : peptide 
174 176 182 186 187
N - Ser Ile Asp Ser Asn Asn Pro Thr Orn Trp Ala Ile Cys Lys - C
Information pour la SEQ ID NO : 10 G1'B
   TYPE DE SEQUENCE : acides aminés
      LONGUEUR DE LA SEQUENCE : 14 acides aminés
      NOMBRES DE BRINS : simple
      CONFIGURATION linéaire
   TYPE DE MOLECULE : peptide 
174 176 182 186 187
N - Ser Ile Asp Gly Asn Asn Gln Leu Orn Lys Ser Ile Cys Lys - C
Information pour la SEQ ID NO : 11 G1'AδC
   TYPE DE SEQUENCE : acides aminés
      LONGUEUR DE LA SEQUENCE : 14 acides aminés
      NOMBRE DE BRINS: simple
      CONFIGURATION : linéaire
   TYPE DE MOLECULE : peptide 
174 176 182 186 187
N - Ser Ile Asp Ser Asn Asn Pro Thr Orn Trp Ala Ile Ser Lys - C
Information pour la SEQ ID NO : 12 Gl'BδC
   TYPE DE SEQUENCE : acides aminés
      LONGUEUR DE LA SEQUENCE : 14 acides aminés
      NOMBRE DE BRINS : simple
      CONFIGURATION : linéaire
   TYPE DE MOLECULE : peptide 
174   176                    182          186 187
N - Ser Ile Asp Gly Asn Asn Gln Leu Orn Lys Ser Ile Ser Lys - C
Information pour la SEQ ID N0 : 13 P40
   TYPE DE SEQUENCE : acides aminés et nucléotides
      LONGUEUR DE LA SEQUENCE : 335 acides aminés, 1005 nucléotides
      NOMBRE DE BRINS : simple
      CONFIGURATION : linéaire
   TYPE DE MOLECULE : protéine 
P40 = N -Ala Pro Lys Asp Asn Thr Trp Tyr Ala Gly Gly Lys Leu Gly Trp Ser
GCT CCG AAA GAT AAC ACC TGG TAT GCA GGT GGT AAA CTG GGT TGG TCC
17
Gln Tyr His Asp Thr Gly Phe Tyr Gly Asn Gly Phe Gln Asn Asn Asn Gly Pro
CAG TAT CAC GAC ACC GGT TTC TAC GGT AAC GGT TTC CAG AAC AAC AAC GGT CCG
35
Thr Arg Asn Asp Gln Leu Gly Ala Gly Ala Phe Gly Gly Tyr Gln Val Asn Pro
ACC CGT AAC GAT CAG CTT GGT GCT GGT GCG TTC GGT GGT TAC CAG GTT AAC CCG
53
Tyr Leu Gly Phe Glu Met Gly Tyr Asp Trp Leu Gly Arg Met Ala Tyr Lys Gly
TAC CTC GGT TTC GAA ATG GGT TAT GAC TGG CTG GGC CGT ATG GCA TAT AAA GGC
71
Ser Val Asp Asn Gly Ala Phe Lys Ala Gln Gly Val Gln Leu Thr Ala Lys Leu
AGC GTT GAC AAC GGT GCT TTC AAA GCT CAG GGC GTT CAG CTG ACC GCT AAA CTG
89
Gly Tyr Pro Ile Thr Asp Asp Leu Asp Ile Tyr Thr Arg Leu Gly Gly Met Val
GGT TAC CCG ATC ACT GAC GAT CTG GAC ATC TAC ACC CGT CTG GGC GGC ATG GTT
107
Trp Arg Ala Asp Ser Lys Gly Asn Tyr Ala Ser Thr Gly Val Ser Arg Ser Glu
TGG CGC GCT GAC TCC AAA GGC AAC TAC GCT TCT ACC GGC GTT TCC CGT AGC GAA
125
His Asp Thr Gly Val Ser Pro Val Phe Ala Gly Gly Val Glu Trp Ala Val Thr
CAC GAC ACT GGC GTT TCC CCA GTA TTT GCT GGC GGC GTA GAG TGG GCT GTT ACT
143
Arg Asp Ile Ala Thr Arg Leu Glu Tyr Gln Trp Val Asn Asn Ile Gly Asp Ala
CGT GAC ATC GCT ACC CGT CTG GAA TAC CAG TGG GTT AAC AAC ATC GGC GAC GCG

161
Gly Thr Val Gly Thr Arg Pro Asp Asn Gly Met Leu Ser Leu Gly Val Ser Tyr
GGC ACT GTG GGT ACC CGT CCT GAT AAC GGC ATG CTG AGC CTG GGC GTT TCC TAC
179
Arg Phe Gly Gln Glu Asp Ala Ala Pro Val Val Ala Pro Ala Pro Ala Pro Ala
CGC TTC GGT CAG GAA GAT GCT GCA CCG GTT GTT GCT CCG GCT CCG GCT CCG GCT
 
197
Pro Glu Val Ala Thr Lys His Phe Thr Leu Lys Ser Asp Val Leu Phe Asn Phe
CCG GAA GTG GCT ACC AAG CAC TTC ACC CTG AAG TCT GAC GTT CTG TTC AAC TTC
215
Asn Lys Ala Thr Leu Lys Pro Glu Gly Gln Gln Ala Leu Asp Gln Leu Tyr Thr
AAC AAA GCT ACC CTG AAA CCG GAA GGT CAG CAG GCT CTG GAT CAG CTG TAC ACT
233
Gln Leu Ser Asn Met Asp Pro Lys Asp Gly Ser Ala Val Val Leu Gly Tyr Thr
CAG CTG AGC AAC ATG GAT CCG AAA GAC GGT TCC GCT GTT GTT CTG GGC TAC ACC
251
Asp Arg Ile Gly Ser Glu Ala Tyr Asn Gln Gln Leu Ser Glu Lys Arg Ala Gln
GAC CGC ATC GGT TCC GAA GCT TAC AAC CAG CAG CTG TCT GAG AAA CGT GCT CAG
269
Ser Val Val Asp Tyr Leu Val Ala Lys Gly Ile Pro Ala Gly Lys Ile Ser Ala
TCC GTT GTT GAC TAC CTG GTT GCT AAA GGC ATC CCG GCT GGC AAA ATC TCC GCT
287
Arg Gly Met Gly Glu Ser Asn Pro Val Thr Gly Asn Thr Cys Asp Asn Val Lys
CGC GGC ATG GGT GAA TCC AAC CCG GTT ACT GGC AAC ACC TGT GAC AAC GTG AAA
305
Ala Arg Ala Ala Leu Ile Asp Cys Leu Ala Pro Asp Arg Arg Val Glu Ile Glu
GCT CGC GCT GCC CTG ATC GAT TGC CTG GCT CCG GAT CGT CGT GTA GAG ATC GAA
323 335
Val Lys Gly Tyr Lys Glu Val Val Thr Gln Pro Ala Gly TER - C
GTT AAA GGC TAC AAA GAA GTT GTA ACT CAG CCG GCG GGT TAA - 3'
Information pour la SEQ ID NO: 14 G2AδCF
   TYPE DE SEQUENCE : acides aminés et nucléotides
      LONGUEUR DE LA SEQUENCE : 101 acides aminés, 303 nucléotides
      NOMBRE DE BRINS : simple
      CONFIGURATION : linéaire
   TYPE DE MOLECULE : protéine 
130
N - Thr Val Lys Thr Lys Asn Thr Thr Thr Thr Gln Thr Gln Pro Ser Lys Pro Thr Thr Lys
5'- ACC GTG AAA ACC AAA AAC ACC ACG ACC ACC CAG ACC CAG CCG AGC AAA CCG ACC ACC AAA
150 163 165 168 170
Gln Arg Gln Asn Lys Pro Pro Asn Lys Pro Asn Asn Asp Ser His Ser Glu Val Ser Asn Ser
CAG CGT CAG AAC AAA CCG CCG AAC AAA CCG AAC AAC GAT TCC CAT TCC GAA GTG TCC AAC TCC
171 173 176 182 186
Val Pro Ser Ser Ile Cys Ser Asn Asn Pro Thr Cys Trp Ala Ile Ser Lys Arg Ile Pro Asn
GTG CCG AGC AGC ATC TGC AGC AAC AAC CCG ACC TGC TGG GCG ATC AGC AAA CGT ATC CCG AAC
192
Lys Lys Pro Gly Lys Lys Thr Thr Thr Lys Pro Thr Lys Lys Pro Thr Phe Lys Thr Thr Lys
AAA AAA CCG GGC AAA AAA ACC ACG ACC AAA CCG ACC AAA AAA CCG ACC TTC AAA ACC ACC AAA
 
213                                                    230
Lys Asp His Lys Pro Gln Thr Thr Lys Pro Lys Glu Val Pro Thr Thr Lys Pro - C
AAA GAT CAT AAA CCG CAG ACC ACC AAA CCG AAA GAA GTG CCG ACC ACC AAA CCG - 3'
Information pour la SEQ ID NO : 15 G4A
   TYPE DE SEQUENCE : acides aminés et nucléotides
      LONGUEUR DE LA SEQUENCE : 17 acides aminés, 42 nucléotides
      NOMBRE DE BRINS : simple
      CONFIGURATION : linéaire
   TYPE DE MOLECULE : peptide 
171 173 176 182 186 187
N - Val Pro Cys Ser Ile Cys Ser Asn Asn Pro Thr Cys Trp Ala Ile Cys Lys - C
5'- GTG CCG TGC AGC ATC TGC AGC AAC AAC CCG ACC TGC TGG GCG ATC TGC AAA - 3'
INFORMATION pour la SEQ ID NO: 16 GAAδC
   TYPE DE SEQUENCE: acides aminés et nucléotides
      LONGUEUR DE LA SEQUENCE : 17 acides aminés 42 nucléotides
      NOMBRE DE BRINS : simple
      CONFIGURATION : linéaire
   TYPE DE MOLECULE : peptide 
171 173 176 182 186 187
N - Val Pro Ser Ser Ile Cys Ser Asn Asn Pro Thr Cys Trp Ala Ile Ser Lys - C
5'- GTG CCG AGC AGC ATC TGC AGC AAC AAC CCG ACC TGC TGG GCG ATC AGC AAA - 3'
Information pour la SEQ ID NO : 17 G4B
   TYPE DE SEQUENCE : acides aminés et nucléotides
      LONGUEUR DE LA SEQUENCE : 17 acides aminés, 42 nucléotides
      NOMBRE DE BRINS : simple
      CONFIGURATION : linéaire
   TYPE DE MOLECULE : peptide 
171 173 176 182 186 187
N - Val Pro Cys Ser Ile Cys Gly Asn Asn Gln Leu Cys Lys Ser Ile Cys Lys - C
5' -GTG CCC TGC AGC ATC TGC GGC AAC AAC CAG CTG TGC AAA AGC ATC TGC AAA - 3'
Information pour la SEQ ID NO : 18 GABδC
   TYPE DE SEQUENCE : acides aminés et nucléotides
      LONGUEUR DE LA SEQUENCE : 17 acides aminés, 42 nucléotides
      NOMBRE DE BRINS : simple
      CONFIGURATION : linéaire
   TYPE DE MOLECULE : peptide 
171 173 176 182 186 187
N - Val Pro Ser Ser Ile Cys Gly Asn Asn Gln Leu Cys Lys Ser Ile Ser Lys - C
5' -GTG CCC AGC AGC ATC TGC GGC AAC AAC CAG CTG TGC AAA AGC ATC AGC AAA - 3'
Information pour la SEQ ID N0 : 19 G4'A
   TYPE DE SEQUENCE : acides aminés
      LONGUEUR DE LA SEQUENCE : 17 acides aminés
      NOMBRE DE BRINS : simple
      CONFIGURATION : Linéaire
   TYPE DE MOLECULE : peptide 
171 173 176 182 186 187
N - Val Pro Asp Ser Ile Asp Ser Asn Asn Pro Thr Orn Trp Ala Ile Orn Lys - C
Information pour la SEQ ID NO : 20 G4'AδC
   TYPE DE SEQUENCE: acides aminés
      LONGUEUR DE LA SEQUENCE : 17 acides aminés
      NOMBRE DE BRINS : simple
      CONFIGURATION : linéaire
   TYPE DE MOLECULE : peptide 
171 173 176 182 186 187
N - Val Pro Ser Ser Ile Asp Ser Asn Asn Pro Thr Orn Trp Ala Ile Ser Lys - C
Information pour la SEQ ID NO : 21 G4'B
   TYPE DE SEQUENCE : acides aminés
      LONGUEUR DE LA SEQUENCE : 17 acides aminés
      NOMBRE DE BRINS : simple
      CONFIGURATION : linéaire
   TYPE DE MOLECULE : peptide 
171 173 176 182 186 187
N - Val Pro Asp Ser Ile Asp Gly Asn Asn Gln Leu Orn Lys Ser Ile Orn Lys - C
Information pour la SEQ ID NO : 22 G4'BδC
   TYPE DE SEQUENCE : acides aminés
      LONGUEUR DE LA SEQUENCE : 17 acides aminés
      NOMBRE DE BRINS : simple
      CONFIGURATION : linéaire
   TYPE DE MOLECULE :peptide 
171 173 176 182 186 187
N - Val Pro Ser Ser Ile Asp Gly Asn Asn Gln Leu Orn Lys Ser Ile Ser Lys - C
Information pour la SEQ ID NO : 23 G200A
   TYPE DE SEQUENCE : acides aminés et nucléotides
      LONGUER DE LA SEQUENCE : 61 acides aminés, 183 nucléotides
      NOMBRE DE BRINS :simple
      CONFIGURATION : linéaire
   TYPE DE MOLECULE : protéine 
140
N - Gln Thr Gln Pro Ser Lys Pro Thr Thr Lys Gln Arg Gln Asn Lys Pro Pro Asn
5'- CAG ACC CAG CCG AGC AAA CCG ACC ACC AAA CAG CGT CAG AAC AAA CCG CCG AAC
158 173 176
Lys Pro Asn Asn Asp Phe His Phe Glu Val Phe Asn Phe Val Pro Cys Ser Ile Cys
AAA CCG AAC AAC GAT TTC CAT TTC GAA GTG TTC AAC TTC GTG CCG TGC AGC ATC TGC
177 182 186
Ser Asn Asn Pro Thr Cys Trp Ala Ile Cys Lys Arg Ile Pro Asn Lys Lys Pro Gly
AGC AAC AAC CCG ACC TGC TGG GCG ATC TGC AAA CGT ATC CCG AAC AAA AAA CCG GGC
196 200
Lys Lys Thr Thr Thr - C
AAA AAA ACC ACG ACC - 3'
Information pour la SEQ ID NO : 24 G198A
   TYPE DE SEQUENCE :acides aminés et nucléotides
      LONGUEUR DE LA SEQUENCE : 59 acides aminés, 177 nucléotides
      NOMBRE DE BRINS : simple
      CONFIGURATION : linéaire
   TYPE DE MOLECULE : protéine 
140
N - Gln Thr Gln Pro Ser Lys Pro Thr Thr Lys Gln Arg Gln Asn Lys Pro Pro Asn
5'- CAG ACC CAG CCG AGC AAA CCG ACC ACC AAA CAG CGT CAG AAC AAA CCG CCG AAC
158                                                                173         176
Lys Pro Asn Asn Asp Phe His Phe Glu Val Phe Asn Phe Val Pro Cys Ser Ile Cys
AAA CCG AAC AAC GAT TTC CAT TTC GAA GTG TTC AAC TTC GTG CCG TGC AGC ATC TGC
177                 182         186
Ser Asn Asn Pro Thr Cys Trp Ala Ile Cys Lys Arg Ile Pro Asn Lys Lys Pro Gly
AGC AAC AAC CCG ACC TGC TGG GCG ATC TGC AAA CGT ATC CCG AAC AAA AAA CCG GGC
196     198
Lys Lys Thr - C
AAA AAA ACC - 3'
Information pour la SEQ ID NO : 25 G196A
   TYPE DE SEQUENCE: acides aminés et nucléotides
      LONGUEUR DE LA SEQUENCE :57 acides aminés, 171 nucléotides
      NOMBRE DE BRINS : simple
      CONFIGURATION : linéaire
   TYPE DE MOLECULE : protéine 
140
N - Gln Thr Gln Pro Ser Lys Pro Thr Thr Lys Gln Arg Gln Asn Lys Pro Pro Asn
5'- CAG ACC CAG CCG AGC AAA CCG ACC ACC AAA CAG CGT CAG AAC AAA CCG CCG AAC
158 173 176
Lys Pro Asn Asn Asp Phe His Phe Glu Val Phe Asn Phe Val Pro Cys Ser Ile Cys
AAA CCG AAC AAC GAT TTC CAT TTC GAA GTG TTC AAC TTC GTG CCG TGC AGC ATC TGC
177 182 186
Ser Asn Asn Pro Thr Cys Trp Ala Ile Cys Lys Arg Ile Pro Asn Lys Lys Pro Gly
AGC AAC AAC CCG ACC TGC TGG GCG ATC TGC AAA CGT ATC CCG AAC AAA AAA CCG GGC
196
Lys - C
AAA - 3'
Information pour la SEQ ID NO : 26 G194A
   TYPE DE SEQUENCE : acides aminés et nucléotides
      LONGUEUR DE LA SEQUENCE : 55 acides aminés, 165 nucléotides
      NOMBRE DE BRINS : simple
      CONFIGURATION : linéaire
   TYPE DE MOLECULE : protéine 
140
N - Gln Thr Gln Pro Ser Lys Pro Thr Thr Lys Gln Arg Gln Asn Lys Pro Pro Asn
5'- CAG ACC CAG CCG AGC AAA CCG ACC ACC AAA CAG CGT CAG AAC AAA CCG CCG AAC
158                                                                173         176
Lys Pro Asn Asn Asp Phe His Phe Glu Val Phe Asn Phe Val Pro Cys Ser Ile Cys
AAA CCG AAC AAC GAT TTC CAT TTC GAA GTG TTC AAC TTC GTG CCG TGC AGC ATC TGC
177             182             186                             194
Ser Asn Asn Pro Thr Cys Trp Ala Ile Cys Lys Arg Ile Pro Asn Lys Lys Pro - C
AGC AAC AAC CCG ACC TGC TGG GCG ATC TGC AAA CGT ATC CCG AAC AAA AAA CCG - 3'
Information pour la SEQ ID NO : 27 G192A
   TYPE DE SEQUENCE : acides aminés et nucléotides
      LONGUEUR DE LA SEQUENCE : 52 acides aminés, 156 nucléotides
      NOMBRE DE BRINS : simple
      CONFIGURATION : linéaire
   TYPE DE MOLECULE : protéine 
140
N - Gln Thr Gln Pro Ser Lys Pro Thr Thr Lys Gln Arg Gln Asn Lys Pro Pro Asn
5'- CAG ACC CAG CCG AGC AAA CCG ACC ACC AAA CAG CGT CAG AAC AAA CCG CCG AAC
158 173 176
Lys Pro Asn Asn Asp Phe His Phe Glu Val Phe Asn Phe Val Pro Cys Ser Ile Cys
AAA CCG AAC AAC GAT TTC CAT TTC GAA GTG TTC AAC TTC GTG CCG TGC AGC ATC TGC
177 182 186 192
Ser Asn Asn Pro Thr Cys Trp Ala Ile Cys Lys Arg Ile Pro Asn Lys - C
AGC AAC AAC CCG ACC TGC TGG GCG ATC TGC AAA CGT ATC CCG AAC AAA - 3'
Information pour la SEQ ID NO : 28 Q6A
   TYPE DE SEQUENCE : acides aminés et nucléotides
      LONGUEUR DE LA SEQUENCE : 51 acides aminés, 153 nucléotides
      NOMBRE DE BRINS : simple
      CONFIGURATION : linéaire
   TYPE DE MOLECULE : protéine 
140
N - Gln Thr Gln Pro Ser Lys Pro Thr Thr Lys Gln Arg Gln Asn Lys Pro Pro Asn
5'- CAG ACC CAG CCG AGC AAA CCG ACC ACC AAA CAG CGT CAG AAC AAA CCG CCG AAC
158 173 176
Lys Pro Asn Asn Asp Phe His Phe Glu Val Phe Asn Phe Val Pro Cys Ser Ile Cys
AAA CCG AAC AAC GAT TTC CAT TTC GAA GTG TTC AAC TTC GTG CCG TGC AGC ATC TGC
177 182 186 190
Ser Asn Asn Pro Thr Cys Trp Ala Ile Cys Lys Arg Ile Pro - C
AGC AAC AAC CCG ACC TGC TGG GCG ATC TGC AAA CGT ATC CCG - 3'
Information pour la SEQ ID NO : 29 G7A
   TYPE LE SEQUENCE : acides aminés et nucléotides
      LONGUEUR DE LA SEQUENCE : 33 acides aminés 99 nucléotides
      NOMBRE DE BRINS : simple
      CONFIGURATION : linéaire
   TYPE DE MOLECULE : protéine 
158 173
N - Lys Pro Asn Asn Asp Phe His Phe Glu Val Phe Asn Phe Val Pro Cys Ser Ile
5'- AAA CCG AAC AAC GAT TTC CAT TTC GAA GTG TTC AAC TTC GTG CCG TGC AGC ATC
176 182 186 190
Cys Ser Asn Asn Pro Thr Cys Trp Ala Ile Cys Lys Arg Ile Pro - C
TGC AGC AAC AAC CCG ACC TGC TGG GCG ATC TGC AAA CGT ATC CCG - 3'
Information pour la SEQ ID NO :30 G200AδC
   TYPE DE SEQUENCE : acides aminés et nucléotides
      LONGUEUR DE LA SEQUENCE : 61 acides aminés, 183 nucléotides
      NOMBRE DE BRINS : simple
      CONFIGURATION : linéaire
      TYPE DE MOLECULE : protéine 
140
N - Gln Thr Gln Pro Ser Lys Pro Thr Thr Lys Gln Arg Gln Asn Lys Pro Pro Asn
5'- CAG ACC CAG CCG AGC AAA CCG ACC ACC AAA CAG CGT CAG AAC AAA CCG CCG AAC
158 173 176
Lys Pro Asn Asn Asp Phe His Phe Glu Val Phe Asn Phe Val Pro Ser Ser Ile Cys
AAA CCG AAC AAC GAT TTC CAT TTC GAA GTG TTC AAC TTC GTG CCG AGC AGC ATC TGC
177 182 186
Ser Asn Asn Pro Thr Cys Trp Ala Ile Ser Lys Arg Ile Pro Asn Lys Lys Pro Gly
AGC AAC AAC CCG ACC TGC TGG GCG ATC AGC AAA CGT ATC CCG AAC AAA AAA CCG GGC
196 200
Lys Lys Thr Thr Thr - C
AAA AAA ACC ACG ACC - 3'
Information pour la SEQ ID NO : 31 G198AδC
   TYPE DE SEQUENCE : acides aminés et nucléotides
      LONGUEUR DE LA SEQUENCE : 59 acides aminés, 177 nucléotides
      NOMBRE DE BRINS : simple
      CONFIGURATION : linéaire
   TYPE DE MOLECULE : protéine 
140
N - Gln Thr Gln Pro Ser Lys Pro Thr Thr Lys Gln Arg Gln Asn Lys Pro Pro Asn
5'- CAG ACC CAG CCG AGC AAA CCG ACC ACC AAA CAG CGT CAG AAC AAA CCG CCG AAC
158 173 176
Lys Pro Asn Asn Asp Phe His Phe Glu Val Phe Asn Phe Val Pro Ser Ser Ile Cys
AAA CCG AAC AAC GAT TTC CAT TTC GAA GTG TTC AAC TTC GTG CCG AGC AGC ATC TGC
177 182 186
Ser Asn Asn Pro Thr Cys Trp Ala Ile Ser Lys Arg Ile Pro Asn Lys Lys Pro Gly
AGC AAC AAC CCG ACC TGC TGG GCG ATC AGC AAA CGT ATC CCG AAC AAA AAA CCG GGC
196 198
Lys Lys Thr - C
AAA AAA ACC - 3'
Information pour la SEQ ID NO : 32 G196AδC
   TYPE DE SEQUENCE : acides aminés et nucléotides
      LONGUEUR DE LA SEQUENCE : 57 acides aminés, 171 nucléotides
      NOMBRE DE BRINS : simple
      CONFIGURATION : linéaire
   TYPE DE MOLECULE : protéine 
140
N - Gln Thr Gln Pro Ser Lys Pro Thr Thr Lys Gln Arg Gln Asn Lys Pro Pro Asn
5'- CAG ACC CAG CCG AGC AAA CCG ACC ACC AAA CAG CGT CAG AAC AAA CCG CCG AAC
 
158                                                                                    173          176
Lys Pro Asn Asn Asp Phe His Phe Glu Val Phe Asn Phe Val Pro Ser Ser Ile Cys
AAA CCG AAC AAC GAT TTC CAT TTC GAA GTG TTC AAC TTC GTG CCG AGC AGC ATC TGC
177                    182                186
Ser Asn Asn Pro Thr Cys Trp Ala Ile Ser Lys Arg Ile Pro Asn Lys Lys Pro Gly
AGC AAC AAC CCG ACC TGC TGG GCG ATC AGC AAA CGT ATC CCG AAC AAA AAA CCG GGC
196
Lys - C
AAA - 3'
Information pour la SEQ ID NO : 33 G194AδC
   TYPE DE SEQUENCE : acides aminés et nucléotides
      LONGUEUR DE LA SEQUENCE : 55 acides aminés, 165 nucléotides
      NOMBRE DE BRINS : simple
      CONFIGURATION : linéaire
   TYPE DE MOLECULE : protéine 
140
N - Gln Thr Gln Pro Ser Lys Pro Thr Thr Lys Gln Arg Gln Asn Lys Pro Pro Asn
5'- CAG ACC CAG CCG AGC AAA CCG ACC ACC AAA CAG CGT CAG AAC AAA CCG CCG AAC
158 173 176
Lys Pro Asn Asn Asp Phe His Phe Glu Val Phe Asn Phe Val Pro Ser Ser Ile Cys
AAA CCG AAC AAC GAT TTC CAT TTC GAA GTG TTC AAC TTC GTG CCG AGC AGC ATC TGC
177 182 186 194
Ser Asn Asn Pro Thr Cys Trp Ala Ile Ser Lys Arg Ile Pro Asn Lys Lys Pro - C
AGC AAC AAC CCG ACC TGC TGG GCG ATC AGC AAA CGT ATC CCG AAC AAA AAA CCG - 3'
Information pour la SEQ ID NO : 34 G192AδC
   TYPE DE SEQUENCE : acides aminés et nucléotides
      LONGUEUR DE LA SEQUENCE : 52 acides aminés, 156 nucléotides
      NOMBRE DE BRINS : simple
      CONFIGURATION : linéaire
   TYPE DE MOLECULE : protéine 
140
N - Gln Thr Gln Pro Ser Lys Pro Thr Thr Lys Gln Arg Gln Asn Lys Pro Pro Asn
5'- CAG ACC CAG CCG AGC AAA CCG ACC ACC AAA CAG CGT CAG AAC AAA CCG CCG AAC
 
158 173 176
Lys Pro Asn Asn Asp Phe His Phe Glu Val Phe Asn Phe Val Pro Ser Ser Ile Cys
AAA CCG AAC AAC GAT TTC CAT TTC GAA GTG TTC AAC TTC GTG CCG AGC AGC ATC TGC
177 182 186 192
Ser Asn Asn Pro Thr Cys Trp Ala Ile Ser Lys Arg Ile Pro Asn Lys - C
AGC AAC AAC CCG ACC TGC TGG GCG ATC AGC AAA CGT ATC CCG AAC AAA - 3'
Information pour la SEQ ID NO : 35 G6AδC
   TYPE DE SEQUENCE : acides aminés et nucléotides
      LONGUEUR DE LA SEQUENCE : 50 acides aminés, 150 nucléotides
      NOMBRE DE BRINS : simple
      CONFIGURATION : linéaire
   TYPE DE MOLECULE : protéine 
140
N - Gln Thr Gln Pro Ser Lys Pro Thr Thr Lys Gln Arg Gln Asn Lys Pro Pro Asn
5'- CAG ACC CAG CCG AGC AAA CCG ACC ACC AAA CAG CGT CAG AAC AAA CCG CCG AAC
158 173 176
Lys Pro Asn Asn Asp Phe His Phe Glu Val Phe Asn Phe Val Pro Ser Ser Ile Cys
AAA CCG AAC AAC GAT TTC CAT TTC GAA GTG TTC AAC TTC GTG CCG AGC AGC ATC TGC
177 182 186 190
Ser Asn Asn Pro Thr Cys Trp Ala Ile Ser Lys Arg Ile Pro - C
AGC AAC AAC CCG ACC TGC TGG GCG ATC AGC AAA CGT ATC CCG - 3'
Information pour la SEQ ID NO : 36 G7AδC
   TYPE DE SEQUENCE : acides aminés et nucléotides
      LONGUEUR DE LA SEQUENCE: 33 acides aminés, 99 nucléotides
      NOMBRE DE BRINS : simple
      CONFIGURATION : linéaire
   TYPE DE MOLECULE : protéine 
158                                                                                    173
N - Lys Pro Asn Asn Asp Phe His Phe Glu Val Phe Asn Phe Val Pro Ser Ser Ile
5'- AAA CCG AAC AAC GAT TTC CAT TTC GAA GTG TTC AAC TTC GTG CCG AGC AGC ATC
 
176 182 186 190
Cys Ser Asn Asn Pro Thr Cys Trp Ala Ile Ser Lys Arg Ile Pro - C
TGC AGC AAC AAC CCG ACC TGC TGG GCG ATC AGC AAA CGT ATC CCG - 3'
Information pour la SEQ ID NO : 37 G200B
   TYPE DE SEQUENCE : acides aminés et nucléotides
      LONGUEUR DE LA SEQUENCE : 61 acides aminés, 183 nucléotides
      NOMBRE DE BRINS : simple
      CONFIGURATION :linéaire
   TYPE DE MOLECULE : protéine 
140
N - Ser Thr Gln Thr Asn Lys Pro Ser Thr Lys Ser Arg Ser Lys Asn Pro Pro Lys Lys Pro
5'- AGC ACC CAG ACC AAC AAA CCG AGC ACC AAA AGC CGT AGC AAA AAC CCG CCG AAA AAA CCG
160 173 176
Lys Asp Asp Tyr His Phe Glu Val Phe Asn Phe Val Pro Cys Ser Ile Cys Gly Asn Asn Gln
AAA GAT GAT TAC CAC TTC GAA GTG TTC AAC TTC GTG CCC TGC AGC ATC TGC GGC AAC AAC CAG
182 186 200
Leu Cys Lys Ser Ile Cys Lys Thr Ile Pro Ser Asn Lys Pro Lys Lys Lys Pro Thr Ile- C
CTG TGC AAA AGC ATC TGC AAA ACC ATC CCG AGC AAC AAA CCG AAA AAG AAA CCG ACC ATC- 3'
Information pour la SEQ ID NO : 38 G198B
   TYPE DE SEQUENCE : acides aminés et nucléotides
      LONGUEUR DE LA SEQUENCE : 59 acides aminés, 177 nucléotides
      NOMBRE DE BRINS : simple
      CONFIGURATION : linéaire
   TYPE DE MOLECULE : protéine 
140
N - Ser Thr Gln Thr Asn Lys Pro Ser Thr Lys Ser Arg Ser Lys Asn Pro Pro Lys Lys Pro
5'- AGC ACC CAG ACC AAC AAA CCG AGC ACC AAA AGC CGT AGC AAA AAC CCG CCG AAA AAA CCG
160 173 176
Lys Asp Asp Tyr His Phe Glu Val Phe Asn Phe Val Pro Cys Ser Ile Cys Gly Asn Asn Gln
AAA GAT GAT TAC CAC TTC GAA GTG TTC AAC TTC GTG CCC TGC AGC ATC TGC GGC AAC AAC CAG
182 186 198
Leu Cys Lys Ser Ile Cys Lys Thr Ile Pro Ser Asn Lys Pro Lys Lys Lys Pro - C
CTG TGC AAA AGC ATC TGC AAA ACC ATC CCG AGC AAC AAA CCG AAA AAG AAA CCG - 3'
Information pour la SEQ ID NO: 39 GL96B
   TYPE DE SEQUENCE: acides aminés et nucléotides
      LONGUEUR DE LA SEQUENCE : 57 acides aminés, 171 nucléotides
      NOMBRE DE BRINS : simple
      CONFIGURATION: linéaire
   TYPE DE MOLECULE: protéine 
140
N - Ser Thr Gln Thr Asn Lys Pro Ser Thr Lys Ser Arg Ser Lys Asn Pro Pro Lys Lys Pro
5'- AGC ACC CAG ACC AAC AAA CCG AGC ACC AAA AGC CGT AGC AAA AAC CCG CCG AAA AAA CCG
160 173 176
Lys Asp Asp Tyr His Phe Glu Val Phe Asn Phe Val Pro Cys Ser Ile Cys Gly Asn Asn Gln
AAA GAT GAT TAC CAC TTC GAA GTG TTC AAC TTC GTG CCC TGC AGC ATC TGC GGC AAC AAC CAG
182 186 196
Leu Cys Lys Ser Ile Cys Lys Thr Ile Pro Ser Asn Lys Pro Lys Lys - C
CTG TGC AAA AGC ATC TGC AAA ACC ATC CCG AGC AAC AAA CCG AAA AAG - 3'
Information pour la SEQ ID NO : 40 G194B
   TYPE DE SEQUENCE : acides aminés et nucléotides
      LONGUEUR DE LA SEQUENCE : 55 acides aminés, 165 nucléotides
      NOMBRE DE BRINS : simple
      CONFIGURATION : linéaire
   TYPE DE MOLECULE : protéine 
140
N - Ser Thr Gln Thr Asn Lys Pro Ser Thr Lys Ser Arg Ser Lys Asn Pro Pro Lys Lys Pro
5'- AGC ACC CAG ACC AAC AAA CCG AGC ACC AAA AGC CGT AGC AAA AAC CCG CCG AAA AAA CCG
160 173 176
Lys Asp Asp Tyr His Phe Glu Val Phe Asn Phe Val Pro Cys Ser Ile Cys Gly Asn Asn Gln
AAA GAT GAT TAC CAC TTC GAA GTG TTC AAC TTC GTG CCC TGC AGC ATC TGC GGC AAC AAC CAG
182 186 194
Leu Cys Lys Ser Ile Cys Lys Thr Ile Pro Ser Asn Lys Pro - C
CTG TGC AAA AGC ATC TGC AAA ACC ATC CCG AGC AAC AAA CCG - 3'
Information pour la SEQ ID NO : 41 G192B
   TYPE DE SEQUENCE : acides aminés et nucléotides
      LONGUEUR DE LA SEQUENCE : 53 acides aminés, 159 nucléotides
      NOMBRE DE BRINS : simple
      CONFIGURATION : linéaire
   TYPE DE MOLECULE : protéine 
140
N - Ser Thr Gln Thr Asn Lys Pro Ser Thr Lys Ser Arg Ser Lys Asn Pro Pro Lys Lys Pro
5'- AGC ACC CAG ACC AAC AAA CCG AGC ACC AAA AGC CGT AGC AAA AAC CCG CCG AAA AAA CCG
160                                                                                    173         176
Lys Asp Asp Tyr His Phe Glu Val Phe Asn Phe Val Pro Cys Ser Ile Cys Gly Asn Asn Gln
AAA GAT GAT TAC CAC TTC GAA GTG TTC AAC TTC GTG CCC TGC AGC ATC TGC GGC AAC AAC CAG
    182                186                        192
Leu Cys Lys Ser Ile Cys Lys Thr Ile Pro Ser Asn - C
CTG TGC AAA AGC ATC TGC AAA ACC ATC CCG AGC AAC - 3'
Information pour la SEQ ID NO : 42 G6B
   TYPE DE SEQUENCE : acides aminés et nucléotides
      LONGUEUR DE LA SEQUENCE : 51 acides aminés, 153 nucléotides
      NOMBRE DE BRINS : simple
      CONFIGURATION : linéaire
   TYPE DE MOLECULE : protéine 
140
N - Ser Thr Gln Thr Asn Lys Pro Ser Thr Lys Ser Arg Ser Lys Asn Pro Pro Lys Lys Pro
5'- AGC ACC CAG ACC AAC AAA CCG AGC ACC AAA AGC CGT AGC AAA AAC CCG CCG AAA AAA CCG
160 173 176
Lys Asp Asp Tyr His Phe Glu Val Phe Asn Phe Val Pro Cys Ser Ile Cys Gly Asn Asn Gln
AAA GAT GAT TAC CAC TTC GAA GTG TTC AAC TTC GTG CCC TGC AGC ATC TGC GGC AAC AAC CAG
182 186 190
Leu Cys Lys Ser Ile Cys Lys Thr Ile Pro - C
CTG TGC AAA AGC ATC TGC AAA ACC ATC CCG - 3'
Information pour la SEQ ID NO : 43 G7B
   TYPE DE SEQUENCE : acides aminés et nucléotides
      LONGUEUR DE LA SEQUENCE : 33 acides aminés, 99 nucléotides
      NOMBRE DE BRINS : simple
      CONFIGURATION : Linéaire
   TYPE DE MOLECULE : protéine 
158 173 176
N - Lys Pro Lys Asp Asp Tyr His Phe Glu Val Phe Asn Phe Val Pro Cys Ser Ile Cys Gly
5'- AAA CCG AAA GAT GAT TAC CAC TTC GAA GTG TTC AAC TTC GTG CCC TGC AGC ATC TGC GGC
182 186 190
Asn Asn Gln Leu Cys Lys Ser Ile Cys Lys Thr Ile Pro - C
AAC AAC CAG CTG TGC AAA AGC ATC TGC AAA ACC ATC CCG - 3'
Information pour la SEQ ID NO: 44 G200BdC
   TYPE DE SEQUENCE : acides aminés et nucléotides
      LONGUEUR DE LA SEQUENCE : 61 acides aminés, 183 nucléotides
      NOMBRE DE BRINS : simple
      CONFIGURATION : linéaire
   TYPE DE MOLECULE : protéine 
140
N - Ser Thr Gln Thr Asn Lys Pro Ser Thr Lys Ser Arg Ser Lys Asn Pro Pro Lys Lys Pro
5'- AGC ACC CAG ACC AAC AAA CCG AGC ACC AAA AGC CGT AGC AAA AAC CCG CCG AAA AAA CCG
160 173 176
Lys Asp Asp Tyr His Phe Glu Val Phe Asn Phe Val Pro Ser Ser Ile Cys Gly Asn Asn Gln
AAA GAT GAT TAC CAC TTC GAA GTG TTC AAC TTC GTG CCC AGC AGC ATC TGC GGC AAC AAC CAG
182 186 200
Leu Cys Lys Ser Ile Ser Lys Thr Ile Pro Ser Asn Lys Pro Lys Lys Lys Pro Thr Ile- C
CTG TGC AAA AGC ATC AGC AAA ACC ATC CCG AGC AAC AAA CCG AAA AAG AAA CCG ACC ATC- 3'
Information pour la SEQ ID NO : 45 GL98BδC
   TYPE DE SEQUENCE : acides aminés et nucléotides
      LONGUEUR DE LA SEQUENCE: 59 acides aminés, 177 nucléotides
      NOMBRE DE BRINS : simple
      CONFIGURATION : Linéaire
   TYPE DE MOLECULE : protéine 
140
N - Ser Thr Gln Thr Asn Lys Pro Ser Thr Lys Ser Arg Ser Lys Asn Pro Pro Lys Lys Pro
5'- AGC ACC CAG ACC AAC AAA CCG AGC ACC AAA AGC CGT AGC AAA AAC CCG CCG AAA AAA CCG
160 173 176
Lys Asp Asp Tyr His Phe Glu Val Phe Asn Phe Val Pro Ser Ser Ile Cys Gly Asn Asn Gln
AAA GAT GAT TAC CAC TTC GAA GTG TTC AAC TTC GTG CCC AGC AGC ATC TGC GGC AAC AAC CAG
182 186 198
Leu Cys Lys Ser Ile Ser Lys Thr Ile Pro Ser Asn Lys Pro Lys Lys Lys Pro - C
CTG TGC AAA AGC ATC AGC AAA ACC ATC CCG AGC AAC AAA CCG AAA AAG AAA CCG - 3'
Information pour la SEQ ID NO : 46 GL96BδC
   TYPE DE SEQUENCE : acides aminés et nucléotides
      LONGUEUR DE LA SEQUENCE: 57 acides aminés, 171 nucléotides
      NOMBRE DE BRINS : simple
      CONFIGURATION: linéaire
   TYPE DE MOLECULE : protéine 
140
N - Ser Thr Gln Thr Asn Lys Pro Ser Thr Lys Ser Arg Ser Lys Asn Pro Pro Lys Lys Pro
5'- AGC ACC CAG ACC AAC AAA CCG AGC ACC AAA AGC CGT AGC AAA AAC CCG CCG AAA AAA CCG
160 173 176
Lys Asp Asp Tyr His Phe Glu Val Phe Asn Phe Val Pro Ser Ser Ile Cys Gly Asn Asn Gln
AAA GAT GAT TAC CAC TTC GAA GTG TTC AAC TTC GTG CCC AGC AGC ATC TGC GGC AAC AAC CAG
182 186 196
Leu Cys Lys Ser Ile Ser Lys Thr Ile Pro Ser Asn Lys Pro Lys Lys - C
CTG TGC AAA AGC ATC AGC AAA ACC ATC CCG AGC AAC AAA CCG AAA AAG - 3'
Information pour la SEQ ID NO : GL94BδC
   TYPE DE SEQUENCE : acides aminés et nucléotides
      LONGUEUR DE LA SEQUENCE : 55 acides aminés, 165 nucléotides
      NOMBRE DE BRINS : simple
      CONFIGURATION : linéaire
   TYPE DE MOLECULE : protéine 
140
N - Ser Thr Gln Thr Asn Lys Pro Ser Thr Lys Ser Arg Ser Lys Asn Pro Pro Lys Lys Pro
5'- AGC ACC CAG ACC AAC AAA CCG AGC ACC AAA AGC CGT AGC AAA AAC CCG CCG AAA AAA CCG
160 173 176
Lys Asp Asp Tyr His Phe Glu Val Phe Asn Phe Val Pro Ser Ser Ile Cys Gly Asn Asn Gln
AAA GAT GAT TAC CAC TTC GAA GTG TTC AAC TTC GTG CCC AGC AGC ATC TGC GGC AAC AAC CAG
182 186 194
Leu Cys Lys Ser Ile Ser Lys Thr Ile Pro Ser Asn Lys Pro - C
CTG TGC AAA AGC ATC AGC AAA ACC ATC CCG AGC AAC AAA CCG - 3'
Information pour la SEQ ID NO : 48 G192BδC
   TYPE DE SEQUENCE : acides aminés et nucléotides
      LONGUEUR DE LA SEQUENCE : 53 acides aminés, 159 nucléotides
      NOMBRE DE BRINS : simple
      CONFIGURATION : linéaire
   TYPE DE MOLECULE : protéine 
140
N - Ser Thr Gln Thr Asn Lys Pro Ser Thr Lys Ser Arg Ser Lys Asn Pro Pro Lys Lys Pro
5'- AGC ACC CAG ACC AAC AAA CCG AGC ACC AAA AGC CGT AGC AAA AAC CCG CCG AAA AAA CCG
160 173 176
Lys Asp Asp Tyr His Phe Glu Val Phe Asn Phe Val Pro Ser Ser Ile Cys Gly Asn Asn Gln
AAA GAT GAT TAC CAC TTC GAA GTG TTC AAC TTC GTG CCC AGC AGC ATC TGC GGC AAC AAC CAG
182 186 192
Leu Cys Lys Ser Ile Ser Lys Thr Ile Pro Ser Asn - C
CTG TGC AAA AGC ATC AGC AAA ACC ATC CCG AGC AAC - 3'
Information pour la SEQ ID NO : 49 G6BδC
   TYPE DE SEQUENCE : acides aminés et nucléotides
      LONGUEUR DE LA SEQUENCE : 51 acides aminés, 153 nucléotides
      NOMBRE DE BRINS : simple
      CONFIGURATION : linéaire
   TYPE DE MOLECULE : protéine 
140
N - Ser Thr Gln Thr Asn Lys Pro Ser Thr Lys Ser Arg Ser Lys Asn Pro Pro Lys Lys Pro
5'- AGC ACC CAG ACC AAC AAA CCG AGC ACC AAA AGC CGT AGC AAA AAC CCG CCG AAA AAA CCG
160                                                                   173         176
Lys Asp Asp Tyr His Phe Glu Val Phe Asn Phe Val Pro Ser Ser Ile Cys Gly Asn Asn Gln
AAA GAT GAT TAC CAC TTC GAA GTG TTC AAC TTC GTG CCC AGC AGC ATC TGC GGC AAC AAC CAG
    182             186             190
Leu Cys Lys Ser Ile Ser Lys Thr Ile Pro - C
CTG TGC AAA AGC ATC AGC AAA ACC ATC CCG - 3'
Information pour la SEQ ID NO : 50 G7BδC
   TYPE DE SEQUENCE : acides aminés et nucléotides
      LONGUEUR DE LA SEQUENCE : 33 acides aminés, 99 nucléotides
      NOMBRE DE BRINS : simple
      CONFIGURATION : linéaire
   TYPE DE MOLECULE : protéine 
158                                                                                    173          176
N - Lys Pro Lys Asp Asp Tyr His Phe Glu Val Phe Asn Phe Val Pro Ser Ser Ile Cys Gly
5'- AAA CCG AAA GAT GAT TAC CAC TTC GAA GTG TTC AAC TTC GTG CCC AGC AGC ATC TGC GGC
                    182                 186                 190
Asn Asn Gln Leu Cys Lys Ser Ile Ser Lys Thr Ile Pro - C
AAC AAC CAG CTG TGC AAA AGC ATC AGC AAA ACC ATC CCG - 3'
Information pour la SEQ ID NO : 51 G2V
   TYPE DE SEQUENCE : acides aminés et nucléotides
      LONGUEUR DE LA SEQUENCE : 101 acides aminés, 303 nucléotides
      NOMBRE DE BRINS : simple
      CONFIGURATION : linéaire
   TYPE DE MOLECULE : protéine 
130
N - Gln Asn Arg Lys Ile Lys Gly Gln Ser Thr Leu Pro Ala Thr Arg Lys Pro Pro Ile Asn
5'- CAA AAC AGA AAA ATC AAA GGT CAA TCA ACA CTA CCA GCC ACA AGA AAA CCA CCA ATT AAT
150
Pro Ser Gly Ser Ile Pro Pro Glu Asn His Gln Asp His Asn Asn Phe Gln Thr Leu Pro Tyr
CCA TCA GGA AGC ATC CCA CCA GAA AAC CAT CAA GAC CAC AAC AAC TTC CAA ACA CTC CCC TAT
171 173 176 182 186
Val Pro Cys Ser Thr Cys Glu Gly Asn Leu Ala Cys Leu Ser Leu Cys His Ile Glu Thr Glu
GTT CCC TGC AGT ACA TGT GAA GGT AAT CTT GCA TGC TTA TCA CTC TGC CAT ATT GAG ACG GAA
192
Arg Ala Pro Ser Arg Ala Pro Thr Ile Thr Leu Lys Lys Thr Pro Lys Pro Lys Thr Thr Lys
AGA GCA CCA AGC AGA GCA CCA ACA ATC ACC CTC AAA AAG ACA CCA AAA CCA AAA ACC ACA AAA
213 230
Lys Pro Thr Lys Thr Thr Ile His His Arg Thr Ser Pro Glu Thr Lys Leu Gln - C
AAG CCA ACC AAG ACA ACA ATC CAT CAC AGA ACC AGC CCA GAA ACC AAA CTG CAA - 3'
Information pour la SEQ ID NO : 52 G2VδC
   TYPE DE SEQUENCE : acides aminés et nucléotides
      LONGUEUR DE LA SEQUENCE : 101 acides aminés, 303 nucléotides
      NOMBRE DE BRINS : simple
      CONFIGURATION : linéaire
   TYPE DE MOLECULE : protéine 
130
N - Gln Asn Arg Lys Ile Lys Gly Gln Ser Thr Leu Pro Ala Thr Arg Lys Pro Pro Ile Asn
5'- CAA AAC AGA AAA ATC AAA GGT CAA TCA ACA CTA CCA GCC ACA AGA AAA CCA CCA ATT AAT
 
150
Pro Ser Gly Ser Ile Pro Pro Glu Asn His Gln Asp His Asn Asn Phe Gln Thr Leu Pro Tyr
CCA TCA GGA AGC ATC CCA CCA GAA AAC CAT CAA GAC CAC AAC AAC TTC CAA ACA CTC CCC TAT
171 173 176 182 186
Val Pro Ser Ser Thr Cys Glu Gly Asn Leu Ala Cys Leu Ser Leu Ser His Ile Glu Thr Glu
GTT CCC AGC AGT ACA TGT GAA GGT AAT CTT GCA TGC TTA TCA CTC AGC CAT ATT GAG ACG GAA
192
Arg Ala Pro Ser Arg Ala Pro Thr Ile Thr Leu Lys Lys Thr Pro Lys Pro Lys Thr Thr Lys
AGA GCA CCA AGC AGA GCA CCA ACA ATC ACC CTC AAA AAG ACA CCA AAA CCA AAA ACC ACA AAA
213 230
Lys Pro Thr Lys Thr Thr Ile His His Arg Thr Ser Pro Glu Thr Lys Leu Gln - C
AAG CCA ACC AAG ACA ACA ATC CAT CAC AGA ACC AGC CCA GAA ACC AAA CTG CAA - 3'
Information pour la SEQ ID NO : 53 G200V
   TYPE DE SEQUENCE : acides aminés et nucléotides
      LONGEUR DE LA SEQUENCE : 61 acides aminés, 183 nucléotides
      NOMBRE DE BRINS : simple
      CONFIGURATION : linéaire
   TYPE DE MOLECULE : protéine 
140
N - Leu Pro Ala Thr Arg Lys Pro Pro Ile Asn Pro Ser Gly Ser Ile Pro Pro Glu Asn His
5'- CTA CCA GCC ACA AGA AAA CCA CCA ATT AAT CCA TCA GGA AGC ATC CCA CCA GAA AAC CAT
160 173 176
Gln Asp His Asn Asn Phe Gln Thr Leu Pro Tyr Val Pro Cys Ser Thr Cys Glu Gly Asn Leu
CAA GAC CAC AAC AAC TTC CAA ACA CTC CCC TAT GTT CCC TGC AGT ACA TGT GAA GGT AAT CTT
182 186 200
Ala Cys Leu Ser Leu Cys His Ile Glu Thr Glu Arg Ala Pro Ser Arg Ala Pro Thr Ile - C
GCA TGC TTA TCA CTC TGC CAT ATT GAG ACG GAA AGA GCA CCA AGC AGA GCA CCA ACA ATC - 3'
Information pour la SEQ ID NO : 54 Gl98V
   TYPE DE SEQUENCE : acides aminés et nucléotides
      LONGUEUR DE LA SEQUENCE : 59 acides aminés, 177 nucléotides
      NOMBRE DE BRINS : simple
      CONFIGURATION : linéaire
   TYPE DE MOLECULE : protéine 
140
N - Leu Pro Ala Thr Arg Lys Pro Pro Ile Asn Pro Ser Gly Ser Ile Pro Pro Glu Asn His
5'- CTA CCA GCC ACA AGA AAA CCA CCA ATT AAT CCA TCA GGA AGC ATC CCA CCA GAA AAC CAT
160 173 176
Gln Asp His Asn Asn Phe Gln Thr Leu Pro Tyr Val Pro Cys Ser Thr Cys Glu Gly Asn Leu
CAA GAC CAC AAC AAC TTC CAA ACA CTC CCC TAT GTT CCC TGC AGT ACA TGT GAA GGT AAT CTT
182 186 198
Ala Cys Leu Ser Leu Cys His Ile Glu Thr Glu Arg Ala Pro Ser Arg Ala Pro - C
GCA TGC TTA TCA CTC TGC CAT ATT GAG ACG GAA AGA GCA CCA AGC AGA GCA CCA - 3'
Information pour la SEQ ID NO:55 G196V
   TYPE DE SEQUENCE : acides aminés et nucléotides
      LONGUEUR DE LA SEQUENCE : 57 acides aminés, 171 nucléotides
      NOMBRE DE BRINS : simple
      CONFIGURATION: linéaire
   TYPE DE MOLECULE : protéine 
140
N - Leu Pro Ala Thr Arg Lys Pro Pro Ile Asn Pro Ser Gly Ser Ile Pro Pro Glu Asn His
5'- CTA CCA GCC ACA AGA AAA CCA CCA ATT AAT CCA TCA GGA AGC ATC CCA CCA GAA AAC CAT
160 173 176
Gln Asp His Asn Asn Phe Gln Thr Leu Pro Tyr Val Pro Cys Ser Thr Cys Glu Gly Asn Leu
CAA GAC CAC AAC AAC TTC CAA ACA CTC CCC TAT GTT CCC TGC AGT ACA TGT GAA GGT AAT CTT
182 186 196
Ala Cys Leu Ser Leu Cys His Ile Glu Thr Glu Arg Ala Pro Ser Arg - C
GCA TGC TTA TCA CTC TGC CAT ATT GAG ACG GAA AGA GCA CCA AGC AGA - 3'
Information pour la SEQ ID NO : 56 G194V
   TYPE DE SEQUENCE : acide aminés et nucléotides
      LONGUEUR DE LA SEQUENCE : 55 acides aminés, 165 nucléotides
      NOMBRE DE BRINS : simple
      CONFIGURATION : linéaire
   TYPE DE MOLECULE : protéine 
140
N - Leu Pro Ala Thr Arg Lys Pro Pro Ile Asn Pro Ser Gly Ser Ile Pro Pro Glu Asn His
5'- CTA CCA GCC ACA AGA AAA CCA CCA ATT AAT CCA TCA GGA AGC ATC CCA CCA GAA AAC CAT
160 173 176
Gln Asp His Asn Asn Phe Gln Thr Leu Pro Tyr Val Pro Cys Ser Thr Cys Glu Gly Asn Leu
CAA GAC CAC AAC AAC TTC CAA ACA CTC CCC TAT GTT CCC TGC AGT ACA TGT GAA GGT AAT CTT
182 186 194
Ala Cys Leu Ser Leu Cys His Ile Glu Thr Glu Arg Ala Pro - C
GCA TGC TTA TCA CTC TGC CAT ATT GAG ACG GAA AGA GCA CCA - 3'
Information pour la SEQ ID NO : 57 G192V
   TYPE DE SEQUENCE : acides aminés et nucléotides
      LONGUEUR DE LA SEQUENCE : 52 acides aminés, 156 nucléotides
      NOMBRE DE BRINS : simple
      CONFIGURATION : linéaire
   TYPE DE MOLECULE : protéine 
140
N - Leu Pro Ala Thr Arg Lys Pro Pro Ile Asn Pro Ser Gly Ser Ile Pro Pro Glu Asn His
5'- CTA CCA GCC ACA AGA AAA CCA CCA ATT AAT CCA TCA GGA AGC ATC CCA CCA GAA AAC CAT
160 173 176
Gln Asp His Asn Asn Phe Gln Thr Leu Pro Tyr Val Pro Cys Ser Thr Cys Glu Gly Asn Leu
CAA GAC CAC AAC AAC TTC CAA ACA CTC CCC TAT GTT CCC TGC AGT ACA TGT GAA GGT AAT CTT
182 186 192
Ala Cys Leu Ser Leu Cys His Ile Glu Thr Glu Arg - C
GCA TGC TTA TCA CTC TGC CAT ATT GAG ACG GAA AGA - 3'
Information pour la SEQ 2D NO, : 58 G6V
   LONGUEUR DE LA SEQUENCE : 51 acides aminés, 153 nucléotides
      NOMBRE DE BRINS : simple
      CONFIGURATION : Linéaire
   TYPE DE MOLECULE : protéine 
140
N - Leu Pro Ala Thr Arg Lys Pro Pro Ile Asn Pro Ser Gly Ser Ile Pro Pro Glu Asn His
5'- CTA CCA GCC ACA AGA AAA CCA CCA ATT AAT CCA TCA GGA AGC ATC CCA CCA GAA AAC CAT
160 173 176
Gln Asp His Asn Asn Phe Gln Thr Leu Pro Tyr Val Pro Cys Ser Thr Cys Glu Gly Asn Leu
CAA GAC CAC AAC AAC TTC CAA ACA CTC CCC TAT GTT CCC TGC AGT ACA TGT GAA GGT AAT CTT
182 186 190
Ala Cys Leu Ser Leu Cys His Ile Glu Thr - C
GCA TGC TTA TCA CTC TGC CAT ATT GAG ACG - 3'
Information pour la SEQ ID N0 : 59 G7V
   TYPE DE SEQUENCE : acides aminés et nucléotides
      LONGUEUR DE LA SEQUENCE : 33 acides aminés, 99 nucléotides
      NOMBRE DE BRINS : simple
      CONFIGURATION : linéaire
   TYPE DE MOLECULE : protéine 
158 173 176
N - Asn His Gln Asp His Asn Asn Phe Gln Thr Leu Pro Tyr Val Pro Cys Ser Thr Cys
5'- AAC CAT CAA GAC CAC AAC AAC TTC CAA ACA CTC CCC TAT GTT CCC TGC AGT ACA TGT
182 186 190
Glu Gly Asn Leu Ala Cys Leu Ser Leu Cys His Ile Glu Thr - C
GAA GGT AAT CTT GCA TGC TTA TCA CTC TGC CAT ATT GAG ACG - 3'
Information pour la SEQ ID NO : 60 G200VδC
   TYPE DE SEQUENCE : acides aminés et nucléotides
      LONGUEUR DE LA SEQUENCE : 61 acides aminés, 183 nucléotides
      NOMBRE DE BRINS : simple
      CONFIGURATION : linéaire
   TYPE DE MOLECULE : protéine 
140
N - Leu Pro Ala Thr Arg Lys Pro Pro Ile Asn Pro Ser Gly Ser Ile Pro Pro Glu Asn His
5'- CTA CCA GCC ACA AGA AAA CCA CCA ATT AAT CCA TCA GGA AGC ATC CCA CCA GAA AAC CAT
160 173 176
Gln Asp His Asn Asn Phe Gln Thr Leu Pro Tyr Val Pro Ser Ser Thr Cys Glu Gly Asn Leu
CAA GAC CAC AAC AAC TTC CAA ACA CTC CCC TAT GTT CCC AGC AGT ACA TGT GAA GGT AAT CTT
182 186 200
Ala Cys Leu Ser Leu Ser His Ile Glu Thr Glu Arg Ala Pro Ser Arg Ala Pro Thr Ile - C
GCA TGC TTA TCA CTC AGC CAT ATT GAG ACG GAA AGA GCA CCA AGC AGA GCA CCA ACA ATC - 3'
   Information pour la SEQ ID NO : 61 G198VδC
      TYPE DE SEQUENCE : acides aminés et nucléotides
      LONGUEUR DE LA SEQUENCE : 59 acides aminés, 177 nucléotides
      NOMBRE DE BRINS : simple
      CONFIGURATION : linéaire
   TYPE MOLECULE : protéine 
140
N - Leu Pro Ala Thr Arg Lys Pro Pro Ile Asn Pro Ser Gly Ser Ile Pro Pro Glu Asn His
5'- CTA CCA GCC ACA AGA AAA CCA CCA ATT AAT CCA TCA GGA AGC ATC CCA CCA GAA AAC CAT
160 173 176
Gln Asp His Asn Asn Phe Gln Thr Leu Pro Tyr Val Pro Ser Ser Thr Cys Glu Gly Asn Leu
CAA GAC CAC AAC AAC TTC CAA ACA CTC CCC TAT GTT CCC AGC AGT ACA TGT GAA GGT AAT CTT
182 186 198
Ala Cys Leu Ser Leu Ser His Ile Glu Thr Glu Arg Ala Pro Ser Arg Ala Pro - C
GCA TGC TTA TCA CTC AGC CAT ATT GAG ACG GAA AGA GCA CCA AGC AGA GCA CCA - 3'
   Information pour la SEQ ID NO : 62 G196VδC
      TYPE DE SEQUENCE : acides aminés et nucléotides
      LONGUEUR DE LA SEQUENCE : 57 acides aminés 171 nucléotides
      NOMBRE DE BRINS : simple
      CONFIGURATION : linéaire
   TYPE DE MOLECULE : protéine 
140
N - Leu Pro Ala Thr Arg Lys Pro Pro Ile Asn Pro Ser Gly Ser Ile Pro Pro Glu Asn His
5'- CTA CCA GCC ACA AGA AAA CCA CCA ATT AAT CCA TCA GGA AGC ATC CCA CCA GAA AAC CAT
160 173 176
Gln Asp His Asn Asn Phe Gln Thr Leu Pro Tyr Val Pro Ser Ser Thr Cys Glu Gly Asn Leu
CAA GAC CAC AAC AAC TTC CAA ACA CTC CCC TAT GTT CCC AGC AGT ACA TGT GAA GGT AAT CTT
182 186 196
Ala Cys Leu Ser Leu Ser His Ile Glu Thr Glu Arg Ala Pro Ser Arg - C
GCA TGC TTA TCA CTC AGC CAT ATT GAG ACG GAA AGA GCA CCA AGC AGA - 3'
Information pour la SEQ ID NO: 63 G194VδC
   TYPE DE SEQUENCE : acides aminés et nucléotides
      LONGUEUR DE LA SEQUENCE : 55 acides aminés, 165 nucléotides
      NOMBRE DE BRINS: simple
      CONFIGURATION : linéaire
   TYPE DE MOLECULE : protéine 
140
N - Leu Pro Ala Thr Arg Lys Pro Pro Ile Asn Pro Ser Gly Ser Ile Pro Pro Glu Asn His
5'- CTA CCA GCC ACA AGA AAA CCA CCA ATT AAT CCA TCA GGA AGC ATC CCA CCA GAA AAC CAT
160 173 176
Gln Asp His Asn Asn Phe Gln Thr Leu Pro Tyr Val Pro Ser Ser Thr Cys Glu Gly Asn Leu
CAA GAC CAC AAC AAC TTC CAA ACA CTC CCC TAT GTT CCC AGC AGT ACA TGT GAA GGT AAT CTT
182 186 194
Ala Cys Leu Ser Leu Ser His Ile Glu Thr Glu Arg Ala Pro - C
GCA TGC TTA TCA CTC AGC CAT ATT GAG ACG GAA AGA GCA CCA - 3'
Information pour la SEQ ID NO : 64 G192VδC
   TYPE DE SEQUENCE : acides aminés et nucléotides
      LONGUEUR DE LA SEQUENCE : 52 acides aminés, 156 nucléotides
      NOMBRE DE BRINS : simple
      CONFIGURATION : linéaire
   TYPE DE MOLECULE : protéine 
140
N - Leu Pro Ala Thr Arg Lys Pro Pro Ile Asn Pro Ser Gly Ser Ile Pro Pro Glu Asn His
5'- CTA CCA GCC ACA AGA AAA CCA CCA ATT AAT CCA TCA GGA AGC ATC CCA CCA GAA AAC CAT
160 173 176
Gln Asp His Asn Asn Phe Gln Thr Leu Pro Tyr Val Pro Ser Ser Thr Cys Glu Gly Asn Leu
CAA GAC CAC AAC AAC TTC CAA ACA CTC CCC TAT GTT CCC AGC AGT ACA TGT GAA GGT AAT CTT
182 186 192
Ala Cys Leu Ser Leu Ser His Ile Glu Thr Glu Arg - C
GCA TGC TTA TCA CTC AGC CAT ATT GAG ACG GAA AGA - 3'
Information pour la SEQ ID NO : 65 G6VδC
   LONGUEUR DE LA SEQUENCE : 51 acides aminés, 153 nucléotides
      NOMBRE DE BRINS : simple
      CONFIGURATION : linéaire
   TYPE DE MOLECULE : protéine 
140
N - Leu Pro Ala Thr Arg Lys Pro Pro Ile Asn Pro Ser Gly Ser Ile Pro Pro Glu Asn His
5'- CTA CCA GCC ACA AGA AAA CCA CCA ATT AAT CCA TCA GGA AGC ATC CCA CCA GAA AAC CAT
160 173 176
Gln Asp His Asn Asn Phe Gln Thr Leu Pro Tyr Val Pro Ser Ser Thr Cys Glu Gly Asn Leu
CAA GAC CAC AAC AAC TTC CAA ACA CTC CCC TAT GTT CCC AGC AGT ACA TGT GAA GGT AAT CTT
182 186 190
Ala Cys Leu Ser Leu Ser His Ile Glu Thr - C
GCA TGC TTA TCA CTC AGC CAT ATT GAG ACG - 3'
Information pour la SEQ ID NO: 66 G7VδC
   TYPE CE SEQUENCE : acides aminés et nucléotides
      LONGUEUR DE LA SEQUENCE :33 acides aminés, 99 nucléotides
      NOMBRE DE BRINS : simple
      CONFIGURATION : linéaire
   TYPE DE MOLECULE : protéine 
158 173 176
N - Asn His Gln Asp His Asn Asn Phe Gln Thr Leu Pro Tyr Val Pro Ser Ser Thr Cys
5'- AAC CAT CAA GAC CAC AAC AAC TTC CAA ACA CTC CCC TAT GTT CCC AGC AGT ACA TGT
182 186 190
Glu Gly Asn Leu Ala Cys Leu Ser Leu Ser His Ile Glu Thr - C
GAA GGT AAT CTT GCA TGC TTA TCA CTC AGC CAT ATT GAG ACG - 3'
Information pour la SEQ ID NO : 67 G4V
   TYPE DE SEQUENCE : acides aminés et nucléotides
      LONGUEUR DE LA SEQUENCE : 17 acides aminés, 51 nucléotides
      NOMBRE DE BRINS : simple
      CONFIGURATION : linéaire
   TYPE DE MOLECULE : peptide 
171 173 176 182 186 187
N - Val Pro Cys Ser Thr Cys Glu Gly Asn Leu Ala Cys Leu Ser Leu Cys His - C
5'- GTT CCC TGC AGT ACA TGT GAA GGT AAT CTT GCA TGC TTA TCA CTC TGC CAT - 3'
Information pour la SEQ ID NO : 68 G4VδC
   TYPE DE SEQUENCE : acides aminés et nucléotides
      LONGUEUR DE LA SEQUENCE : 17 acides aminés, 51 nucléotides
      NOMBRE DE BRINS :simple
      CONFIGURATION : linéaire
   TYPE DE MOLECULE : peptide 
171 173 176 182 186 187
N - Val Pro Ser Ser Thr Cys Glu Gly Asn Leu Ala Cys Leu Ser Leu Ser His - C
5'- GTT CCC AGC AGT ACA TGT GAA GGT AAT CTT GCA TGC TTA TCA CTC AGC CAT - 3'
Information pour la SEQ ID NO : 69 G4'V
   TYPE DE SEQUENCE : acides aminés
      LONGUEUR DE LA SEQUENCE : 17 acides aminés
      NOMBRE DE BRINS : simple
      CONFIGURATION : linéaire
   TYPE DE MOLECULE : peptide 
171 173 176 182 186 187
N - Val Pro Asp Ser Thr Asp Glu Gly Asn Leu Ala Orn Leu Ser Leu Orn His - C
Information pour la SEQ ID NO : 70 G4'VδC
   TYPE DE SEQUENCE : acides aminés
      LONGUEUR DE LA SEQUENCE : 17 acides aminés
      NOMBRE DE BRINS : simple
      CONFIGURATION : Linéaire
   TYPE DE MOLECULE : peptide 
171 173 176 182 186 187
N - Val Pro Ser Ser Thr Asp Glu Gly Asn Leu Ala Orn Leu Ser Leu Ser His - C
Information pour la SEQ ID NO :71 G1V
   TYPE DE SEQUENCE : acides aminés et nucléotides
      LONGUEUR DE LA SEQUENCE : 14 acides aminés, 42 nucléotides
      NOMBRE DE BRINS : simple
      CONFIGURATION : linéaire
   TYPE DE MOLECULE : peptide 
174 176 182 186 187
N - Ser Thr Cys Glu Gly Asn Leu Ala Cys Leu Ser Leu Cys His - C
5'- AGT ACA TGT GAA GGT AAT CTT GCA TGC TTA TCA CTC TGC CAT - 3'
Information pour la SEQ ID NO : 72 G1VδC
   TYPE DE SEQUENCE : acides aminés et nucléotides
      LONGUEUR DE LA SEQUENCE : 14 acides aminés
      NOMBRE DE BRINS : simple
      CONFIGURATION : linéaire
   TYPE DE MOLECULE : peptide 
174 176 182 186 187
N - Ser Thr Cys Glu Gly Asn Leu Ala Cys Leu Ser Leu Ser His - C
5'- AGT ACA TGT GAA GGT AAT CTT GCA TGC TTA TCA CTC AGC CAT - 3'
Information pour la SEQ ID NO : 73 G1'VδC
   TYPE DE SEQUENCE : acides aminés
      LONGUEUR DE LA SEQUENCE : 14 acides aminés
      NOMBRE DE BRINS : simple
      CONFIGURATION : linéaire
   TYPE DE MOLECULE: peptide 
174 176 182 186 187
N - Ser Thr Asp Glu Gly Asn Leu Ala Orn Leu Ser Leu Ser His - C
Information pour la SEQ ID NO : 74 BB
   TYPE DE SEQUENCE : acides aminés et nucléotides
      LONGUEUR DE LA SEQUENCE : 219 acides aminés, 657 nucléotides
      NOMBRE DE BRINS : simple
      CONFIGURATION : linéaire
   TYPE DE MOLECULE : protéine 
1
BB = N - Lys Tyr Gly Val Ser Asp Tyr Tyr Lys Asn Leu Ile Asn Asn Ala Lys
5'- AAA TAT GGA GTA AGT GAC TAT TAC AAG AAT CTA ATC AAC AAT GCC AAA

17
Thr Val Glu Gly Val Lys Asp Leu Gln Ala Gln Val Val Glu Ser Ala Lys Lys
ACT GTT GAA GGC GTA AAA GAC CTT CAA GCA CAA GTT GTT GAA TCA GCG AAG AAA

35
Ala Arg Ile Ser Glu Ala Thr Asp Gly Leu Ser Asp Phe Leu Lys Ser Gln Thr
GCG CGT ATT TCA GAA GCA ACA GAT GGC TTA TCT GAT TTC TTG AAA TCA CAA ACA

53
Pro Ala Glu Asp Thr Val Lys Ser Ile Glu Leu Ala Glu Ala Lys Val Leu Ala
CCT GCT GAA GAT ACT GTT AAA TCA ATT GAA TTA GCT GAA GCT AAA GTC TTA GCT
 
71
Asn Arg Glu Leu Asp Lys Tyr Gly Val Ser Asp Tyr His Lys Asn Leu Ile Asn
AAC AGA GAA CTT GAC AAA TAT GGA GTA AGT GAC TAT CAC AAG AAC CTA ATC AAC

89
Asn Ala Lys Thr Val Glu Gly Val Lys Asp Leu Gln Ala Gln Val Val Glu Ser
AAT GCC AAA ACT GTT GAA GGT GTA AAA GAC CTT CAA GCA CAA GTT GTT GAA TCA

107
Ala Lys Lys Ala Arg Ile Ser Glu Ala Thr Asp Gly Leu Ser Asp Phe Leu Lys
GCG AAG AAA GCG CGT ATT TCA GAA GCA ACA GAT GGC TTA TCT GAT TTC TTG AAA

125
Ser Gln Thr Pro Ala Glu Asp Thr Val Lys Ser Ile Glu Leu Ala Glu Ala Lys
TCA CAA ACA CCT GCT GAA GAT ACT GTT AAA TCA ATT GAA TTA GCT GAA GCT AAA

143
Val Leu Ala Asn Arg Glu Leu Asp Lys Tyr Gly Val Ser Asp Tyr Tyr Lys Asn
GTC TTA GCT AAC AGA GAA CTT GAC AAA TAT GGA GTA AGT GAC TAT TAC AAG AAC

161
Leu Ile Asn Asn Ala Lys Thr Val Glu Gly Val Lys Ala Leu Ile Asp Glu Ile
CTA ATC AAC AAT GCC AAA ACT GTT GAA GGT GTA AAA GCA CTG ATA GAT GAA ATT

179
Leu Ala Ala Leu Pro Lys Thr Asp Thr Tyr Lys Leu Ile Leu Asn Gly Lys Thr
TTA GCT GCA TTA CCT AAG ACT GAC ACT TAC AAA TTA ATC CTT AAT GGT AAA ACA

197
Leu Lys Gly Glu Thr Thr Thr Glu Ala Val Asp Ala Ala Thr Ala Arg Ser Phe
TTG AAA GGC GAA ACA ACT ACT GAA GCT GTT GAT GCT GCT ACT GCA AGA TCT TTC

215 219
Asn Phe Pro Ile Leu - C
AAT TTC CCT ATC CTC - 3'
Information pour la SEQ ID NO : 75 δBE = fragment de EB
   TYPE DE SEQUENCE : acides aminés et nucléotides
      LONGUEUR DE LA SEQUENCE : 108 acides aminés, 324 nucléotides
      NOMBRE DE BRINS : simple
      CONFIGURATION : linéaire
   TYPE DE MOLECULE : protéine

1
δ28 = N -Lys Tyr Gly Val Ser Asp Tyr His Lys Asn Leu Ile Asn Asn Ala Lys
5'- AAA TAT GGA GTA AGT GAC TAT CAC AAG AAC CTA ATC AAC AAT GCC AAA
17
Thr Val Glu Gly Val Lys Asp Leu Gln Ala Gln Val Val Glu Ser Ala Lys Lys
ACT GTT GAA GGT GTA AAA GAC CTT CAA GCA CAA GTT GTT GAA TCA GCG AAG AAA
35
Ala Arg Ile Ser Glu Ala Thr Asp Gly Leu Ser Asp Phe Leu Lys Ser Gln Thr
GCG CGT ATT TCA GAA GCA ACA GAT GGC TTA TCT GAT TTC TTG AAA TCA CAA ACA
53
Pro Ala Glu Asp Thr Val Lys Ser Ile Glu Leu Ala Glu Ala Lys Val Leu Ala
CCT GCT GAA GAT ACT GTT AAA TCA ATT GAA TTA GCT GAA GCT AAA GTC TTA GCT
71
Asn Arg Glu Leu Asp Lys Tyr Gly Val Ser Asp Tyr Tyr Lys Asn Leu Ile Asn
AAC AGA GAA CTT GAC AAA TAT GGA GTA AGT GAC TAT TAC AAG AAC CTA ATC AAC
89
Asn Ala Lys Thr Val Glu Gly Val Lys Ala Leu Ile Asp Glu Ile Leu Ala Ala
AAT GCC AAA ACT GTT GAA GGT GTA AAA GCA CTG ATA GAT GAA ATT TTA GCT GCA
107 108
Leu Pro - C
TTA CCT - 3'

## Revendications

1. Protéine- isolée **caractérisée en ce que** ladite protéine est la protéine OmpA de la membrane externe de la bactérie Klebsiella pneumoniae de séquence SEQ ID N° 13.

2. Protéine selon la revendication 1, **caractérisée en ce qu'**elle est obtenue par voie recombinante.

3. Protéine selon la revendication 1, **caractérisée en ce qu'**elle est obtenue par purification à partir d'une culture de Klebsiella pneumoniae.

4. Protéine selon l'une des revendications 1 à 3, **caractérisée en ce que** ladite protéine est une protéine porteuse.

5. Conjugué **caractérisé en ce qu'**il comprend une protéine selon l'une des revendications 1 à 4 et un peptide immunogène, ledit peptide immunogène étant **caractérisé en ce qu'**il comprend la séquence peptidique comprise entre les résidus d'acides aminés 174 et 187 et **en ce qu'**il est porté par la séquence peptidique SEQ ID N° 1, N° 2 ou N° 51 comprise entre les résidus d'acides 130 et 230 de la séquence de la protéine G du virus respiratoire syncytial humain du sous groupe A et B, ou du virus respiratoire syncytial bovin, et dans lequel peptide immunogène l'aminoacide cys en position 173 et/ou 186 de ladite protéine G peut être une sérine.

6. Conjugué selon la revendication 5, **caractérisé en ce qu'**il est obtenu par couplage chimique.

7. Conjugué selon la revendication 5 ou 6, **caractérisé en ce qu'**il est soluble.

8. Conjugué selon la revendication 7, **caractérisé en ce que** le conjugué est obtenu par le procédé comprenant les étapes suivantes :
a) on précipite les lipopolysaccharides de membranes de bactéries du genre Klebsiella, en présence d'un sel de cation divalent et de détergents, pour récupérer les protéines membranaires totales dans le surnageant,
b) on soumet les protéines à une chromatographie par échange d'anions pour séparer la fraction contenant la protéine adjuvante d'immunité,
c) on concentre la fraction contenant la protéine adjuvante d'immunité,
d) on conjugue la protéine adjuvante d'immunité avec un polypeptide immunogène pour former un conjugué soluble,
ledit peptide immunogène étant **caractérisé en ce qu'**il comprend la séquence peptidique comprise entre les résidus d'acides aminés 174 et 187 et **en ce qu'**il est porté par la séquence peptidique SEQ ID N° 1, N° 2 ou N° 51 comprise entre les résidus d'acides 130 et 230 de la séquence de la protéine G du virus respiratoire syncytial humain du sous groupe A et B, ou du virus respiratoire syncytial bovin, et dans lequel peptide immunogène l'aminoacide cys en position 173 et/ou 186 de ladite protéine G peut être une serine.

9. Conjugué selon l'une des revendications 5 à 8, **caractérisé en ce que** ledit peptide immunogène est conjugué à la protéine porteuse par la protéine BB de liaison à la sémmalbumine humaine.

10. Conjugué selon la revendication 9, **caractérisé en ce que** la protéine BB comprend la protéine de séquence SEQ ID N° 74 ou de séquence SEQ ID N° 73.

11. Conjugué selon la revendication 9 ou 10, **caractérisé en ce qu'**il est obtenu par recombinaison génétique.

12. **Acide nucléique isolé caractérisé en ce que sa séquence code pour la protéine de séquence SEQ ID N° 13.**

13. Composition vaccinale **caractérisée en ce qu'**elle comprend un conjugué selon l'une des revendications 5 à 11.

14. Composition selon la revendication 13, administrable par voie injectable ou par voie sous-cutanée.

15. Composition selon la revendication 13 ou 14, comprenant en outre des excipients pharmaceutiquement acceptables.

16. Utilisation d'une protéine selon la revendication 1, pour la préparation d'une composition destinée à la prévention ou le traitement des affections à virus syncytial respiratoire (VRS).

17. Anticorps dirigés contre la protéine de séquence SEQ ID N° 13.

## Claims

1. Isolated protein, **characterized in that** said protein is the OmpA outer membrane protein of the Klebsiella pneumoniae bacterium of sequence SEQ ID No. 13.

2. Protein according to Claim 1, **characterized in that** it is obtained by the recombinant pathway.

3. Protein according to Claim 1, **characterized in that** it is obtained by purification from a culture of Klebsiella pneumoniae.

4. Protein according to one of Claims 1 to 3, **characterized in that** said protein is a carrier protein.

5. Conjugate, **characterized in that** it comprises a protein according to one of Claims 1 to 4 and an immunogenic peptide, said immunogenic peptide being **characterized in that** it comprises the peptide sequence included between amino acid residues 174 and 187, and **in that** it is carried by the peptide sequence SEQ ID No.1, No. 2 and No. 51 included between acid residues 130 and 230 of the sequence of the G protein of the human respiratory syncytial virus subgroups A and B, or of the bovine respiratory syncytial virus, and in which immunogenic peptide the cys amino acid at position 173 and/or 186 of said G protein can be a serine.

6. Conjugate according to Claim 5, **characterized in that** it is obtained by chemical coupling.

7. Conjugate according to Claim 5 or 6, **characterized in that** it is soluble.

8. Conjugate according to Claim 7, **characterized in that** the conjugate is obtained by means of the method comprising the following steps:
a) the membrane lipopolysaccharides of bacteria of the Klebsiella genus are precipitated in the presence of a divalent cation salt and of detergents, so as to recover the total membrane proteins in the supernatant,
b) the proteins are subjected to anion exchange chromatography so as to separate the fraction containing the immune adjuvant protein,
c) the fraction containing the immune adjuvant protein is concentrated,
d) the immune adjuvant protein is conjugated with an immunogenic polypeptide so as to form a soluble conjugate,
said immunogenic peptide being **characterized in that** it comprises the peptide sequence included between amino acid residues 174 and 187, and **in that** it is carried by the peptide sequence SEQ ID No. 1, No. 2 or No. 51 included between acid residues 130 and 230 of the sequence of the G protein of the human respiratory syncytial virus subgroups A and B, or of the bovine respiratory syncytial virus, and in which immunogenic peptide the cys amino acid at position 173 and/or 186 of said G protein can be a serine.

9. Conjugate according to one of Claims 5 to 8, **characterized in that** said immunogenic peptide is conjugated to the carrier protein by means of the human serum albumin-binding BB protein.

10. Conjugate according to Claim 9, **characterized in that** the BB protein comprises the protein of sequence SEQ ID No. 74 or of sequence SEQ ID No. 73.

11. Conjugate according to Claim 9 or 10, **characterized in that** it is obtained by genetic recombination.

12. Isolated nucleic acid, **characterized in that** its sequence encodes the protein of sequence SEQ ID No. 13.

13. Vaccine composition, **characterized in that** it comprises a conjugate according to one of Claims 5 to 11.

14. Composition according to Claim 13, that can be administered by injection or subcutaneously.

15. Composition according to Claim 13 or 14, also comprising pharmaceutically acceptable excipients.

16. Use of a protein according to Claim 1, for preparing a composition for use in the prevention or treatment of respiratory syncytial virus (RSV) conditions.

17. Antibodies directed against the protein of sequence SEQ ID No. 13.

## Patentansprüche

1. Isoliertes Protein, **dadurch gekennzeichnet, dass** das Protein das Protein OmpA der äußeren Membran des Bakteriums Klebsiella pneumoniae mit der Sequenz SEQ ID Nr. 13 ist.

2. Protein nach Anspruch 1, **dadurch gekennzeichnet, dass** es auf rekombinantem Wege erhalten wird.

3. Protein nach Anspruch 1, **dadurch gekennzeichnet, dass** es durch Reinigung ausgehend von einer Kultur von Klebsiella pneumoniae erhalten wird.

4. Protein nach einem der Ansprüche 1 bis 3, **dadurch gekennzeichnet, dass** das Protein ein Trägerprotein ist.

5. Konjugat, **dadurch gekennzeichnet, dass** es ein Protein nach einem der Ansprüche 1 bis 4 und ein immunogenes Peptid umfasst, wobei das immunogene Peptid **dadurch gekennzeichnet ist, dass** es die Peptidsequenz zwischen den Aminosäureresten 174 und 187 eingeschlossen umfasst und dass es durch die Peptidsequenz SEQ lD Nr. 1, Nr. 2 oder Nr. 51, welche zwischen den Säureresten 130 und 230 eingeschlossen der Sequenz des Proteins G des humanen Respiratory-Syncytial-Virus der Untergruppe A und B oder des Respiratory-Syncytial-Virus vom Rind liegt, getragen wird und in welchem immunogenen Peptid die Aminosäure Cys an Position 173 und/oder 186 des Proteins G ein Serin sein kann.

6. Konjugat nach Anspruch 5, **dadurch gekennzeichnet, dass** es durch chemische Kopplung erhalten wird.

7. Konjugat nach Anspruch 5 oder 6, **dadurch gekennzeichnet, dass** es löslich ist.

8. Konjugat nach Anspruch 7, **dadurch gekennzeichnet, dass** das Konjugat erhalten wird durch das Verfahren, umfassend die folgenden Schritte:
a) man fällt die Lipopolysaccharide von Membranen von Bakterien der Gattung Klebsiella in Gegenwart eines Salzes eines zweiwertigen Kations und von Detergentien aus, um die gesamten Membranproteine im Überstand zu gewinnen,
b) man unterwirft die Proteine einer Anionenaustauschchromatographie, um die Fraktion, welche das Immunitäts-Adjuvansprotein enthält, abzutrennen,
c) man konzentriert die Fraktion, welche das Immunitäts-Adjuvansprotein enthält, auf,
d) man konjugiert das Immunitäts-Adjuvansprotein mit einem immunogenen Polypeptid, um ein lösliches Konjugat zu bilden,
wobei das immunogene Polypeptid **dadurch gekennzeichnet ist, dass** es die Peptidsequenz zwischen den Aminosäureresten 174 und 187 eingeschlossen umfasst und dass es durch die Peptidsequenz SEQ ID Nr. 1, Nr. 2 oder Nr. 51, welche zwischen den Säureresten 130 und 230 eingeschlossen der Sequenz des Proteins G des humanen Respiratory-Syncytial-Virus der Untergruppe A und B oder des Respiratory-Syncytial-Virus vom Rind liegt, getragen wird und in welchem immunogenen Peptid die Aminosäure Cys an Position 173 und/oder 186 des Proteins G ein Serin sein kann.

9. Konjugat nach einem der Ansprüche 5 bis 8, **dadurch gekennzeichnet, dass** das immunogene Peptid mit dem Trägerprotein durch das für die Bindung an humanes Serumalbumin zuständige Protein BB konjugiert ist.

10. Konjugat nach Anspruch 9, **dadurch gekennzeichnet, dass** das Protein BB das Protein mit der Sequenz SEQ ID Nr. 74 oder der Sequenz SEQ ID Nr. 73 umfasst.

11. Konjugat nach Anspruch 9 oder 10, **dadurch gekennzeichnet, dass** es durch genetische Rekombination erhalten wird.

12. Isolierte Nukleinsäure, **dadurch gekennzeichnet, dass** deren Sequenz das Protein mit der Sequenz SEQ ID Nr. 13 kodiert.

13. Impfzusammensetzung, **dadurch gekennzeichnet, dass** sie ein Konjugat nach einem der Ansprüche 5 bis 11 umfasst.

14. Zusammensetzung nach Anspruch 13, welche auf dem Injektionsweg oder auf subkutanem Wege verabreichbar ist

15. Zusammensetzung nach Anspruch 13 oder 14, welche außerdem pharmazeutisch annehmbare Trägersubstanzen umfasst.

16. Verwendung eines Proteins nach Anspruch 1 für die Herstellung einer Zusammensetzung, welche für die Verhütung oder die Behandlung von Respiratory-Syncytial-Virus (RSV)-Erkrankungen bestimmt ist.

17. Antikörper, welche gegen das Protein mit der Sequenz SEQ ID Nr. 13 gerichtet sind.
